(19) Europäisches Patentamt
European Patent Office
Office européen des brevets

(11) Veröffentlichungsnummer: **0 178 624**
**B1**

(12) **EUROPÄISCHE PATENTSCHRIFT**

(45) Veröffentlichungstag der Patentschrift: **22.11.90**

(51) Int. Cl.$^5$: **A 61 K 9/50**

(21) Anmeldenummer: **85113027.8**

(22) Anmeldetag: **14.10.85**

(54) **Liposomen aus synthetischen Lipiden.**

(30) Priorität: **16.10.84 CH 4951/84**

(43) Veröffentlichungstag der Anmeldung:
**23.04.86 Patentblatt 86/17**

(45) Bekanntmachung des Hinweises auf die Patenterteilung:
**22.11.90 Patentblatt 90/47**

(84) Benannte Vertragsstaaten:
**AT BE CH DE FR GB IT LI LU NL SE**

(56) Entgegenhaltungen:
**EP-A-0 088 046**
**EP-A-0 099 068**
**EP-A-0 147 741**
**DE-A-2 647 395**
**US-A-4 302 459**
**US-E- 30 748**

**CHEMICAL ABSTRACTS, Band 100, Nr. 25, 18. Juni 1984, Seite 220, Zusammenfassung Nr. 205314k, Columbus, Ohio, US; P. VAN HOOGEVEST et al.: "The influence of lipid composition on glycophorin-induced bilayer permeability"**

(73) Patentinhaber: **CIBA-GEIGY AG**
**Klybeckstrasse 141**
**CH-4002 Basel (CH)**

(72) Erfinder: **Tarcsay, Lajos, Dr.**
**Muttenzerstrasse 25**
**D-7889 Grenzbach-Wyhlen (DE)**
Erfinder: **Eibl, Hansjörg, Prof. Dr.**
**Steinweg 51**
**D-3406 Bovenden (DE)**
Erfinder: **Fankhauser, Peter, Dr.**
**Hauptstrasse 65**
**CH-4107 Ettingen (CH)**
Erfinder: **Peil, Anita, Dr.**
**Feldbergstrasse 50**
**CH-4057 Basel (CH)**

(74) Vertreter: **Zumstein, Fritz, Dr. et al**
**Bräuhausstrasse 4**
**D-8000 München 2 (DE)**

**EP 0 178 624 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Gegenstand der vorliegenden Erfindung sind pharmazeutische Zusammensetzungen enthaltend synthetische, zu mehr als 90 Gew% reine Phospholipide und einen pharmazeutischen Wirkstoff, Gemische aus synthetischen, im wesentlichen reinen Phospholipiden, Verfahren zur Herstellung dieser pharmazeutischen Zusammensetzungen bzw. Gemische, sowie deren Verwendung.

Die erfindungsgemässen pharmazeutischen Zusammensetzungen werden in Form von Liposomen in wässriger Dispersion angewendet.

Liposomen sind in der Literatur in zahlreichen Veröffentlichungen beschrieben worden. Ihre Aufbau und ihre Verwendung sind Gegenstand vieler Untersuchungen. Man unterscheidet unilamellare Liposomen mit einer Doppelschicht aus Lipiden von multilamellaren Liposomen mit mehreren Doppelschichten aus Lipiden, die zwiebelschalenförmig angeordnet sind.

Für die pharmazeutische Anwendung eignen sich insbesondere Liposomen mit einer Population von möglichst homogener Grösse und einem Durchmesser von ca. $2{,}0 \times 10^{-8}$ bis $5{,}0 \times 10^{-6}$ m, vorzugsweise ca. $2{,}0 \times 10^{-8}$ bis $3{,}0 \times 10^{-6}$ m. Die kugelförmige Hülle besteht aus einer oder mehreren Doppelschichten der Lipidkomponenten, z.B. amphipatischen Lipiden, z.B. Phospholiden, z.B. Lecithin, Kephalin oder Phosphatidsäure, und gegebenenfalls neutralen Lipiden, z.B. Cholesterin. Diese Doppelschichten umschliessen einen Innenraum, der die wässrige Phase mit einer einzuschliessenden Verbindung enthält, wobei die einzuschliessende Verbindung abhängig von ihrer Struktur und weiteren Parametern wie Temperatur oder Konzentration in der wässrigen Phase und/oder in der Doppelschicht vorhanden sein kann.

Es besteht grosses Interesse an der therapeutischen Verwendung von Liposomen als Träger von Wirkstoffen unterschliedlichster Art. So sind Liposomen als Träger von Proteinen z.B. Antikörpern oder Enzymen, Hormonen, Vitaminen oder Genen oder zu analytischen Zwecken als Träger von markierten Verbindungen vorgeschlagen worden.

Pharmazeutische Verabreichungssysteme auf Liposomenbasis sind in dem Uebersichtswerk von Gregoriadis G. (Herausgeber) Liposome Technology, Vol. II, Incorporation of Drugs, Proteins and Genetic Material, CRC Press 1984, beschrieben. Im Uebersichtswerk von Knight, C. G. (Herausgeber), Liposomes: From Physical Structure to Therapeutic Applications, Elsevier 1981, sind im Kapital 16 auf S. 166 die Vorteile eine pharmazeutischen Verabreichungsform auf Liposomenbasis zusammengefasst:

1. Liposomen durchdringen biologische Membranen und erleichtern den Transport von Wirkstoffen über normalerweise unüberwindliche Barrieren. Insbesondere erleichtern Liposomen die intrazelluläre Durchdringung mit einer verkapselten Verbindung.

2. Liposomen können im Hinblick auf gezielte Wechselwirkungen mit bestimmten Zellgewebearten, erhöhte Selektivität und verminderte Toxizität eingesetzt werden.

3. Die Pharmakokinetik eines Wirkstoffs kann durch Liposomen vorteilhaft beeinflusst werden, z.B. durch Aenderung der Freisetzung, Verteilung und Entfernung aus der systemischen Zirkulation.

4. Gegenüber Aenderungen durch chemische Einflüsse und Metabolismus empfindliche Wirkstoffe werden durch Liposomen vor Desaktivierung geschützt.

5. Durch Stimulierung von Immunreaktionen auf Liposomen-verkapselte Antigene können immunmodulatorische Effekte erzielt werden.

Daraus ergeben sich weitere Vorteile wie verminderte Wirkstoffmengen, die bei Liposomen-Applikation im Vergleich mit freiem Wirkstoff zur Erzielung eines therapeutischen Effekts benötigt werden oder verlängerte Intervallzeiten für die Verabreichung des Wirkstoffs.

Besondere Vorteile haben Verabreichungssysteme auf Liposomenbasis zur Einschleusung von Stoffen in endocytierende Zellen, insbesondere des Retikuloendothelial-Systems. So beobachtet man beispielsweise eine Erleichterung des Transports von Antibiotika in endocytierende Zellen und eine verbesserte Bekämpfung der in diesen Zellen befindlichen Erreger. Endocytierende Zellen sind auch bei entzündlichen Prozessen beteiligt. Man beobachtet eine schnellere Einschleusung von Liposomenverkapselten antirheumatischen Wirkstoffen in solche Zellen als in das umliegrende Gewebe. In Form von Liposomen verkapselte Zytostatika können in die spezifischen Organe des Retikuloendothelial-System (Leber, Milz, Knochenmark) eingeschleust, oder es können in der Lunge infolge von Filtration in den Lungenkapillaren und anschliessendem Transport durch auswandernde Blutmonocyten Wirkstoffe in alveolären Makrophagen angereichert und damit eine Verbesserung der Wirkung auf Lungen- oder Lebertumore bei gleichzeitiger Verminderung der Toxizität erreicht werden.

Liposomen mit verkapselten Immunmodulatoren können eine gezielte Aenderung der Reaktionen des Immunsystems (Immunstimulation, Immunsuppression) bewirken. So wird von Poste G. et al. in Cancer Research 39, 881 (1979) eine Aktivierung von tumoriciden Eigenschaften von Maus-Makrophagen durch Liposom-verkapselte Lymphokine beobachtet. Sone S. und I. J. Fidler, Cell. Immunol. 57, 42 (1981) berichten von einer in-vitro Aktivierung von tumoriciden Eigenschaften in alveolären Makrophagen von Ratten durch synthetische, in Liposomen verkapselte Muramyldipeptide.

Liposomen mit Immunmodulatoren, z.B. Muramyldi- und Muramyltripeptiden, humanem Gamma-Interferon oder Makrophagen-aktivierendem-Faktor (MAF), eignen sich zur Aktivierung des zellulären Immunsystems, z.B. der Zellen des monocytären Systems, z.B. der Blutmonocyten oder der alveolären oder peritonealen Makrophagen, zur Abwehr von Infektionen, insbesondere Virusinfektionen, und zur

Bekämpfung von Tumorzellen in primärem Tumorgewebe in Blut und Lymphe, sowie von Metastasen.

Das bisher verwendete natürliche Material zur Herstellung von Liposomen, z.B. natürliche Phospholipide, z.B. Ei-Phosphatidsäure, Ei- und Sojalecithin oder Ei- oder Sojakephalin sowie Phosphatidylserin aus dem Rinderhirn, auch wenn dieses Material in gereinigter Form vorliegt und Dünnschicht- oder Papierchromatographisch einheitlich sein sollte, ist ein Gemisch aus Phosphoglyceriden mit Acylresten unterschiedlicher Struktur. Trockenpräparate mit natürlichen Phospholipiden sind thermolabil und nur kurze Zeit haltbar und natürliche Phospholipide in wässriger Phase sind ebenfalls instabil, so dass die Haltbarkeit von wässrigen Liposomenmischungen ebenfalls nur begrenzt ist.

Aufgrund der wechselnden Zusammensetzungen der Liposomenmischungen aus natürlichen Phospholipiden, geringer Ausbeute, unterschiedlicher Grössenverteilung und ihrer geringer Stabilität fehlt den *in-vitro* und *in-vivo* Versuchsergebnissen sowie den klinischen Resultaten die erforderliche Reproduzierbarkeit, was sich bislang hemmend auf die industrielle Verwertbarkeit dieser seit längerem bekannten und intensiv erforschten Darreichungsform ausgewirkt hat.

In der Europäischen Patentanmeldung Publikationsnummer 88 046 ist ein Verfahrfen zur Herstellung von unilamelleren Liposomen unter Verwendung von natürlichen und synthetischen Phospholipiden beschrieben. Wie die Beispiele zeigen, ist die Grössenverteilung der Liposomen unterschiedlich. So werden Fraktionen mit kleinen Liposomen im Grössenbereich von $2{,}0 \times 10^{-8}$ bis $1{,}0 \times 10^{-7}$ m und grossen Liposomen im Grössenbereich bis zu $5{,}0 \times 10^{-6}$ m und unterschiedlicher Mengenverteilung erhalten.

Der vorliegenden Erfindung liegt die Aufgabe zugrunde, pharmazeutische Zusammensetzungen herzustellen, welche in wässriger Phase Liposomendispersionen mit einheitlicher Grössenverteilung und hoher Stabilität bilden.

Die vorliegende Erfindung betrifft pharmazeutische Zusammensetzungen enthaltend

a) ein synthetisches, im wesentlichen reines, Phospholipid der Formel

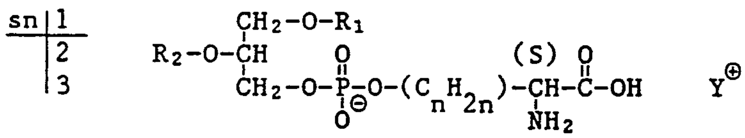

(I),

worin $R_1$ und $R_2$ unabhängig voneinander $C_{10}$—$C_{20}$-Alkenoyl mit gerader Anzahl an C-Atomen, n eine ganze Zahl von eins bis drei und $Y^{\oplus}$ das Kation einer pharmazeutischen annehmbaren Base darstellen,

b) ein synthetisches, im wesentlichen reines, Phospholipid der Formel

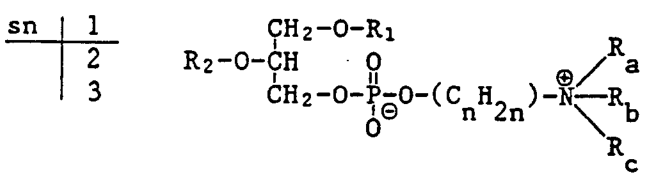

(II),

worin $R_1$ $C_{10}$—$C_{20}$-Alkanoyl mit gerader Anzahl an C-Atomen, $R_2$ $C_{10}$—$C_{20}$-Alkenoyl mit gerader Anzahl an C-Atomen, $R_a$, $R_b$ und $R_c$ Wasserstoff oder $C_1$—$C_4$-Alkyl und n eine ganze Zahl von zwei bis vier darstellen,

c) einen Stoff oder ein Stoffgemisch mit biologischer Wirkung und gegebenenfalls eine Trägerflüsigkeit und/oder zusätzliche feste Trägermaterialien.

Die weiter vorn und im folgenden genannten allgemeinen Begriffe haben im Rahmen der Beschreibung der vorliegenden Erfindung vorzugsweise die folgenden Bedeutungen:

Der im Zusammenhang mit organischen Resten, z.B. Niederalkyl, Niederalkylen, Niederalkoxy, Niederalkanoyl etc., verwendete Ausdruck "Nieder" bedeutet, dass solche organischen Reste, falls nicht ausdrücklich anders definiert, bis einschliesslich 7 und bevorzugt bis einschliesslich 4 Kohlenstoffatome enthalten.

Die Nomenklatur der Phospholipide der Formeln I und II erfolgt anhand der in Eur. J. of Biochem. 79, 11—21 (1977) "Nomenclature of Lipids" von der IUPAC—IUB Commission on Biochemical Nomenclature (CBN) gegeben Empfehlungen (sn-Nomenklatur, stereospecific numbering).

Für die pharmazeutischen Wirkstoffe werden, wenn nicht anders angegeben, die von der Weltgesundheitsorganisation (WHO) vorgeschlagenen Freinamen (Recommended International Nonproprietary Names) verwendet, welche dem Standardwerk "Pharmazeutische Chemie" (E. Schröder, C. Rufer und R. Schmiechen, Thieme Verlag Stuttgart, 1982), sowie dem Merck-Index (Tenth Edition), entnommen wurden.

Die Reinheit der verwendeten synthetischen Phospholipide beträgt mehr als 90 Gewichts-%, vorzugsweise mehr als 95%.

In einem synthetischen Phospholipid der Formel I sind $R_1$ und $R_2$ mit der Bedeutung "$C_{10}$—$C_{20}$-Alkenoyl mit gerader Anzahl an C-Atomen" vorzugsweise 9-cis-Dodecenoyl, 9-cis-Tetradecenoyl, 9-cis-Hexadecenoyl, 6-cis-Octadecenoyl, 6-trans-Octadecenoyl, 9-cis-Octadecenoyl, 9-trans-Octadecenoyl, 11-cis-Octadecenoyl oder 9-cis-Icosenoyl.

Das Kation $Y^{\oplus}$ einer pharmazeutisch annehmbaren Base ist beispielsweise in Alkalimetall-, z.B. das Lithium-, Natrium- oder Kaliumion, das Ammoniumion, ein Mono-, Di- oder Tri-$C_1$—$C_4$-alkylammoniumion, z.B. das Trimethyl-, Aethyl-, Diäthyl-oder Triäthylammoniumion, ein 2-Hydroxyäthyl-tri-$C_1$—$C_4$-alkylammoniumion, z.B. das Cholinkation, oder das 2-Hydroxyäthylammoniumion, sowie das Kation einer basischen Aminosäure, z.B. Lysin oder Arginin.

$Y^{\oplus}$ ist bevorzugt das Natriumion.

In einem synthetischen Phospholipid der Formel I sind vorzugsweise $R_1$ und $R_2$ gleich und bedeuten 9-cis-Dodecanoyl, 9-cis-Tetradecenoyl, 9-ci-Hexadecenoyl, 9-cis-Octadecenoyl oder 9-cis-Icosenoyl, n ist eins und $Y^{\oplus}$ ist das Natriumion.

Ein synthetisches Phospholipid der Formel I ist in erster Linie Natrium-1,2-di-(9-cis-octadecenoyl)-3-sn-phosphatidyl-(S)-serin.

In einem synthetischen Phospholipid der Formel II ist $R_1$ mit der Bedeutung "$C_{10}$—$C_{20}$-Alkanoyl mit gerader Anzahl an C-Atomen" vorzugsweise n-Dodecanoyl, n-Tetradecanoyl, n-Hexadecanoyl, n-Octadecanoyl oder n-Icosanoyl.

In einem synthetischen Phospholipid der Formel II hat $R_2$ die unter Formel I genannten Bedeutungen.

In einem synthetischen Phospholipid der Formel II ist die Gruppe der Formel —$(C_nH_{2n})$— unverzweigtes oder verzweigtes Alkylen, z.B. 1,1-Aethylen, 1,1-, 1,2- oder 1,2-Propylen oder 1,2-, 1,3- oder 1,4-Butylen, oder vorzugsweise 1,2-Aethylen (n=2).

In einem synthetischen Phospholipid der Formel II bedeuten vorzugsweise $R_1$ n-Dodecanoyl, n-Tetradecanoyl, n-Hexadecanoyl oder n-Octadecanoyl, $R_2$ 9-cis-Dodecenoyl, 9-cis-Tetradecenoyl, 9-cis-Hexadecenoyl, 9-cis-Octadecenoyl oder 9-cis-Icosenoyl und $R_a$, $R_b$ und $R_c$ Methyl und n ist zwei.

Ein synthetisches Phospholipid der Formel II ist in erster Linie 1-n-Hexadecanoyl-2-(9-cis-octadecenoyl)-3-an-phosphatidylcholin.

Für die Acylreste $R_1$ und $R_2$ in den Phospholipiden der Formel I und II sind die in Klammern angegebenen Bezeichnungen gebrächlich:

9-cis-Dodecenoyl (Lauroleoyl), 9-cis-Tetradecenoyl (Myristoleoyl), 9-cis-Hexadecenoyl (Palmitoleoyl), 6-cis-Octadecenoyl (Petroseloyl), 6-trans-Octadecenoyl (Petroselaidoyl), 9-cis-Octadecenoyl (Oleoyl), 9-trans-Octadecenoyl (Elaidoyl), 11-cis-Octadecenoyl (Vaccenoyl), 9-cis-Icosenoyl (Gadoleoyl), n-Dodecanoyl (Lauroyl), n-Tetradecanoyl (Myristoyl), n-Hexadecanoyl (Palmitoyl), n-Octadecanoyl (Stearoyl), n-Icosanoyl (Arachidoyl).

Stoffe oder Stoffgemische mit biologischer Wirkung sind in erster Linie pharmazeutische Wirkstoffe und Stoffgemische aus der Gruppe der Antiphlogistika, Antibiotika, Antileishmanien, Antimykotika, Antineoplastika und Immunmodulatoren.

Pharmazeutische Wirkstoffe aus der Gruppe der Antiphlogistica sind z.B. Glucocorticoide, z.B. Cortison, Hydrocortison, Prednison, Prednisolon, Fluocortolon, Triamcinolon, Methylprednisolon, Prednyliden, Paramethason, Dexamethason, Betamethason, Beclomethason, Fluprednyliden, Desoximethason, Fluocinolon, Flumethason, Diflucortolon, Clocortolon, Clobetasol oder Fluocortinbutylester, nicht-steroide Entzündungshemmer aus der Gruppe der substituierten Phenylessigsäuresalze oder 2-Phenylpropionsäuresalze, z.B. Alclofenac, Ibufenac, Ibuprofen, Clindanac, Fanclorac, Ketoprofen, Fenoprofen, Indoprofen, Fenclofenac, Diclofenac, Flurbiprofen, Pirprofen, Naproxen, Benoxaprofen, Carprofen oder Cicloprofen, Anthransilsäure-Derivate, z.B. der Formel

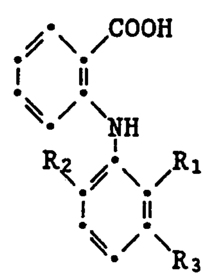

(III),

worin $R_1$, $R_2$ und $R_3$ unabhängig voneinander Wasserstoff, Methyl, Chlor oder Trifluormethyl bedeuten, z.B. Mefenaminsäure, Flufenaminsäure, Tolfenaminsäure oder Meclofenaminsäure, Anilino-substituierte Nicotinsäure-Derivate, z.B. Mifluminsäure, Clonixin oder Flunixin, Heteroarylessigsäuren oder 2-Heterarylessigsäuren mit einem 2-Indol-3-yl- oder Pyrrol-2-yl-Rest, z.B. Indometacin, Oxmetacin, Intrazol, Acemetazin, Cinmetacin, Zomepirac, Tolmetin, Colpirac oder Tiaprofensäure, eine Indenylessigsäure vom Sulindac-Typ, sowie analgetisch wirksame Heteroaryloxyessigsäuren, z.B. Benzadac.

Pharmazeutische Wirkstoffe aus der Gruppe der Antibiotika sind z.B. Tetracyclin-Antibiotika der Formel

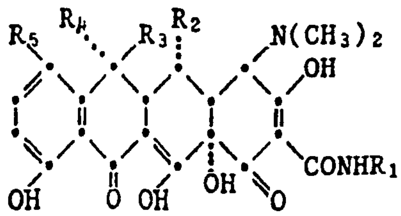

(V),

worin $R_1$ Wasserstoff oder Pyrrolidin-1-ylmethyl, $R_2$ Wasserstoff oder Hydroxy, $R_3$ Wasserstoff, Hydroxy oder Methyl, $R_4$ Wasserstoff oder Methyl und $R_5$ Wasserstoff, Chlor oder Dimethylamino bedeuten, z.B. Chlortetracyclin, Oxytetracyclin, Tetracyclin, Demethylchlortetracyclin, Metacyclin, Doxycyclin, Minocyclin oder Rolitetracyclin, Aminoglycoside, z.B. Kanamycin, Amikacin, Gentamycin $C_{1a}$, $C_2$, $C_{2b}$ oder $C_1$, Sisomicin, Netilmicin, Spectinomycin, Streptomycin, Tobramycin, Neomycin B, Dibekacin oder Kanendomycin, Makrolide, z.B. Maridomycin oder Erythromycin, Lincomycine, z.B. Clindamycin oder Lincomycin, Penicillansäure (6—APA)- und Cephalosporansäure (7—ACA)-Derivate mit 6β bzw. 7β-Acylaminogruppen, welche in fermentativ, halb- oder totalsynthetisch erhältlichen 6β-Acylaminopenicillansäure- oder 7β-Acylaminocephalosporansäurederivaten oder in 3-Stellung abgewandelten 7β-Acylaminocephalosporansäurederivaten vorhanden sind, z.B. unter den Namen Penicillin G oder V, Phenethicillin, Propicillin, Nafcillin, Oxacillin, Cloxazillin, Dicloxacillin, Flucloxacillin, Cylcazillin, Epicillin, Mecillinam, Methicillin, Azlocillin, Sulbenicillin, Ticarcillin, Mezlocillin, Piperacillin, Carindacillin, Azidocillin oder Ciclazillin bekannt gewordene Penicillansäurederivate oder unter den Namen Cefaclor, Cefuroxim, Cefazlur, Cephacetril, Cefazolin, Cephalexin, Cefadroxil, Cephaloglycin, Cefoxitin, Cephaloridin, Cefsulodin, Cefotiam, Ceftazidin, Cefonicid, Cefotaxim, Cefmenoxim, Ceftizoxim, Cephalothin, Cephradin, Cefamandol, Cephapirin, Cefroxadin, Cefatrizin, Cefazedon, Ceftrixon oder Ceforanid bekannt gewordene Cephalosporinderivate, sowie andere β-Lactam-Antibiotica vom Clavam-, Penem- oder Carbapenem-Typ, z.B. Moxalactam, Clavulansäure, Nocardicin A, Sulbactam, Aztreonam oder Thienamycin, sowie Antibiotika vom Bicozamycin-, Novobiocin-, Chlor- oder Thiamphenicol-, Rifampicin-, Fosfomycin-, Colistin- oder Vancomycin-Typ.

Pharmazeutische Wirkstoffe aus der Gruppe der Antileishmanien sind z.B. Antimon-Verbindungen, z.B. Brechweinstein (Kalium-antimonyltartrat), Stibopens, Natriumstibocaptat sowie Natriumstibogluconat.

Pharmazeutische Wirkstoffe aus der Gruppe der Antimykotika sind z.B. Thiokohlensäurederivatge, z.B. Dibenzthion, Tolnaftat oder Tolciclat, Imidazol-Derivate, z.B. Clotrimazol, Miconazol, Econazol, Isoconazol oder Ketoconazol oder Polyen-Antibiotika, z.B. Nystatin, Natamycin oder Amphotericin B.

Pharmazeutische Wirkstoffe aus der Gruppe Antineoplastika sind beispielsweise Alkylanzien mit Bis-(2-chloräthyl)-amin-Gruppe, z.B. Chlormethin, Chlorambucil, Melphalan, Uramustin, Mannomustin, Estramustinphosphat, Mechlorethaminoxid, Cyclophosphamid, Ifosfamid oder Trifosfamid, Alkylanzien mit Aziridin-Struktur, z.B. Tretamin, Thiotepa, Triaziquon oder Mitomycin, alkylierende Methansulfonsäureester, z.B. Busulfan, alkylierende N-Alkyl-N-nitrosoharnstoffderivate, z.B. Carmustin, Lomustin, Semustin oder Streptozotocin, sowie Alkylanzien vom Mitobronitol-, Dacarbazin- oder Procarbazin-Typ, Antimetabolite vom Folsäure-Typ, z.B. Methotrexat, Purin-Derivate, z.B. Mercaptopurin, Thioguanin, Azathioprin, Thiamiprin, Vidarabin oder Puromycin, Pyrimidin-Derivate, z.B. Fluorouracil, Floxuridin, Tegafur, Cytarabin, Idoxuridin, Flucytosin, Antibiotika, welche in der Krebschemotherapie eingesetzt werden, z.B. Dactinomycin, Daunorubicin, Doxorubicin, Mithramycin, Bleomycin $A_2$ oder $B_2$ oder Etoposid, sowie Vinca-Alkaloide, z.B. Vincristin, gegebenenfalls in Kombination mit Chlormethamin, Prednisolon oder Prednison und Procarbazin.

Immunmodulatoren sind z.B. Muramylpeptide, z.B. Muramyldipeptide oder Muramyltripeptide, insbesondere der Formel

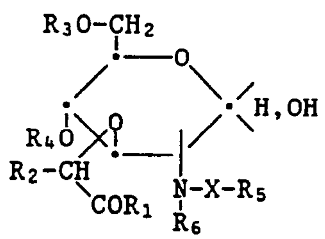

(VI),

worin X die Gruppen —C(=O)— oder —C(=O)—O—, $R_1$ die L - Ala - D - isoGln - L - Ala - 2 - (1,2 - - dipalmitoyl - sn - glycero - 3 - hydroxyphosphoryloxy) - äthylamid, L - Ala - D - Glu(Cy - L - Ala - 2 - (1,2 - dipalmitoyl - sn - glycero - 3 - hydroxyphosphoryloxy) - äthylamid)-, L - Ala - D - isoGlnOH-, L - Ala - D - isoGlnNH$_2$ - α - n - butylester-, L - Ala - D - isoGln - L - (stearoyl) - Lys-, L - Val - D - Gln - NH$_2$ - α - n - methylester-, L - Ala - D - isoGln - L - Ala - 1,2 - dipalmitoyl - sn - glycerinester oder die L - Ala - D - isoGln - L - Ala - cholesterinester - Gruppe, $R_2$ Wasserstoff, Methyl oder n-Propyl, $R_3$

Wasserstoff, n-Stearoyl, 10 - (2,3 - Dimethoxy - 1,4 - dioxo - 5 - methyl) - 2,5 - cyclohexadienoyl, 2 - Behenoyloxy - 2 - methylpropanoyl oder n-Octanoyl, $R_4$ Wasserstoff oder n-Octanoyl bedeuten, $R_5$ $C_1$—$C_4$-Alkyl und $R_6$ Wasserstoff oder $C_1$—$C_4$-Alkyl sowie die entsprechenden 2 - Palmitoylthio - Derivate davon, Lipopeptide mit immunmodulatorischen Eigenschaften vom Typ n - Lauroyl - L - Ala - D - isoGln - (m - DAP - Gly) - $NH_2$, n - Lauroyl - L - Ala - D - isoGln - (L - DAP - Gly) - $NH_2$, n - Lauroyl - L - Ala - D - isoGln - (L - Lys - D - Ala) - $NH_2$, n - Octanoyl - L - Ala - D - isoGln - (L - Lys - D - Ala) - $NH_2$ oder Palmitoyl - Cys - (2R) - 2,3 - dilauroyloxypropyl) - Ala - D - Glu(Gly - taurin - Na) - - $NH_2$, oder sind Lymphokine, die von Lymphozyten, Monozyten oder Makrophagen ausgeschieden werden, wenn diese durch Antigene oder Mitogene oder dergleichen stimuliert werden.

Zur Gruppe der Lymphokine gehören beispielsweise bekannte Interferon-Typen, insbesondere natürliches oder rekombiniertes, humanes Gamma-Interferon, z.B. humanes Gamma-Interferon, das gemäss den Europäischen Patentanmeldungen 63,482, 77,670, 83,777, 88,540, 89,676, 95,350, 99,084, 110,044 und 112,967 und den Internationalen Anmeldungen (PCT) WO 83/04,053 und WO 84/02,129 erhältlich ist.

Bevorzugt ist rekombiniertes humanes Gamma-Interferon gemäss Europäischer Patentanmeldung 121,157 mit folgender Aminosäuresequenz:

$H_2N$-Cys-Tyr-Cys-Gln-Asp-Pro-Tyr-Val-Gln-Glu-Ala-Glu-Asn-Leu-Lys-Lys-
Tyr-Phe-Asn-Ala-Gly-His-Ser-Asp-Val-Ala-Asp-Asn-Gly-Thr-Leu-Phe-Leu-
Gly-Ile-Leu-Lys-Asn-Trp-Lys-Glu-Glu-Ser-Asp-Arg-Lys-Ile-Met-Gln-Ser-
Gln-Ile-Val-Ser-Phe-Tyr-Phe-Lys-Leu-Phe-Lys-Asn-Phe-Lys-Asp-Asp-Gln-
Ser-Ile-Gln-Lys-Ser-Val-Glu-Thr-Ile-Lys-Glu-Asp-Met-Asn-Val-Lys-Phe-
Phe-Asn-Ser-Asn-Lys-Lys-Lys-Arg-Asp-Asp-Phe-Glu-Lys-Leu-Thr-Asn-Tyr-
Ser-Val-Thr-Asp-Leu-Asn-Val-Gln-Arg-Lys-Ala-Ile-His-Glu-Leu-Ile-Gln-
Val-Met-Ala-Glu-Leu-Ser-Pro-Ala-Ala-Lys-Thr-Glu-Lys-Arg-Lys-Arg-Ser-
Gln-Met-Leu-Phe-Gln-Gly-Arg-Arg-Ala-Ser-Gln-OH,

und rekombiniertes humanes Gamma-Interferon gemäss Britischer Patentanmeldung 2,107,718 mit folgender Aminosäuresequenz:

$H_2N$-Cys-Tyr-Cys-Gln-Asp-Pro-Tyr-Val-Lys-Glu-Ala-
Glu-Asn-Leu-Lys-Lys-Tyr-Phe-Asn-Ala-Gly-His-Ser-
Asp-Val-Ala-Asp-Asn-Gly-Thr-Leu-Phe-Leu-Gly-Ile-
Leu-Lys-Asn-Trp-Lys-Glu-Glu-Ser-Asp-Arg-Lys-Ile-
Met-Gln-Ser-Gln-Ile-Val-Ser-Phe-Tyr-Phe-Lys-Leu-
Phe-Lys-Asn-Phe-Lys-Asp-Asp-Gln-Ser-Ile-Gln-Lys-
Ser-Val-Glu-Thr-Ile-Lys-Glu-Asp-Met-Asn-Val-Lys-
Phe-Phe-Asn-Ser-Asn-Lys-Lys-Lys-Arg-Asp-Asp-Phe-
Glu-Lys-Leu-Thr-Asn-Tyr-Ser-Val-Thr-Asp-Leu-Asn-
Val-Gln-Arg-Lys-Ala-Ile-His-Glu-Leu-Ile-Gln-Val-
Met-Ala-Glu-Leu-Ser-Pro-Ala-Ala-Lys-Thr-Gly-Lys-
Arg-Lys-Arg-Ser-Gln-Met-Leu-Phe-Arg-Gly-Arg-Arg-
Ala-Ser-Gln-OH.

Zur gruppe der Lymphokine gehört ausserdem in gereinigter Form vorliegendes, humanes Interleukin 2, z.B. nach Aktivierung von humanen, neoplastischen Leukämie- oder Lymphom-Zellen durch T-Zellmitogen im Kulturfiltrat erhältliches und durch Umkehrphasen-HPLC-gereinigtes Interleukin 2, aus Kulturen mit menschlichen T-Lymphozyten aus Milz oder peripherem Blut nach Stimulierung durch

Antigene oder Mitogene, z.B. humane T-Zellen-Leukämie-Lymphom-Viren (HTLV—I) Phytohämagglutinin oder Concanavalin A, erhältliche Kulturfiltrate, welche Gemische mit den unter den Begriffen Makrophagen-Migration-Inhibitionsfaktor (MIF), Leukozyten-Migration-Inhibitionsfaktor, Leukocyten-Migration-Verstärkungsfaktor, Makrophagen-Aktivierender-Faktor (MAF), Kolonien-stimulierender-Faktor, Interleukin 1 und 2 sowie Gamma-Interferon bekannt gewordenen Bestandteilen enthalten, insbesondere solche Kulturfiltrate oder Isolate mit einem hohen Anteil an Makrophagen-Aktivierendem-Faktor (MAF).

Die erfindungsgemässen pharmazeutischen Zusammensetzungen zeichnen sich durch relativ einheitliche Grösse (ca. 2,0—3,0 × $10^{-9}$m) und hohe Lagerstabilität aus. So sind beispielsweise Trockenpräparate (Lyophilisate) bestehend aus den genannten synthetischen Phospholipiden und Muramylpeptiden mehrere Monate bis zu mehrere Jahre haltbar. Die Trockenpräparate lassen sich kurz von Gebrauch auf einfachste Weise in wässriger Pufferlösung durch Schütteln (Vortex) oder Vibrieren dispergieren und in situ applizieren, wobei in den meisten Fällen Zusatzmassnahmen wie Filtration, Neutralisation, Dialyse, etc. vermieden werden können. Wässrige Liposomendispersionen mit synthetischen Phospholipiden und Muramylpeptiden als Einschlussverbindung sind bei Temperaturen unter 10°C mehrere Wochen bis Monate haltbar und können sogar als Lyophilisat lagerstabil gemacht werden.

Die erfindungsgemässen pharmazeutischen Zusammensetzungen zeichnen sich durch besonders gute physiologische Verträglichkeit z.B. geringe Toxizität, und günstiges pharmakokinetisches Profil aus, wenn sie in Form einer wässrigen Dispersion von Liposomen verabreicht werden. So erfolgt sehr rasche Endocytose, insbesondere durch die Zellen des monocytären Systems. Liposomen bestehend aus den genannten synthetischen Phospholipiden und einem Muramyldi- oder Muramyltripeptid als Einschlussverbindung lassen sich besonders gut in der Lunge und Leber anreichern und werden von Makrophagen rasch endocytiert. Insbesondere werden alevoläre Makrophagen stimuliert und physiologisch abartige Materialien, z.B. Viren oder metastasierende Tumorzellen, beseitigt. Daher eignen sich die erfindungsgemässen pharmazeutischen Zusammensetzungen in Form von Liposomen in besonders hohem Mass in der Krebschemotherapie zur Bekämpfung von metastasierenden Tumoren.

Das zur Herstellung der erfindungsgemässen pharmazeutischen Zusammensetzungen verwendbare Gemisch der Phospholipide (I) und (II) hat nach Dispersion in wässriger Phase eine Phasenübergangstemperatur (flüssig-gelförmig) niedriger als ca. 37°C. Die Herstellung der Liposomendispersion kann ohne Erwärmen erfolgen.

Wässrige Dispersionen, worin die Phospholipide der Formeln I und II in Form von Liposomen vorliegen, worin die genannten Verbindungen bzw. Stoffgemische mit biologischer Wirkung eingeschlossen sind, sind pharmazeutische Verabreichungssysteme, welche sich, gegebenenfalls nach Konzentrierung oder Isolierung der Liposomen, z.B. durch Ultrazentrifugieren, zu therapeutischen Zwecken für die orale (p.o.) oder parenterale (i.v., i.m., i.p. oder topisch) Verabreichung eignen.

Bei oraler Verabreichtung können Verabreichungssysteme auf Liposomenbasis die Resorption eines Wirkstoffs verbessern.

Für die orale Verabreichung kann die Liposomen-haltige wässrige Dispersion, abgepuffert auf pH 7,0—7,8, vorzugsweise 7,2—7,4, mit pharmazeutisch unbedenklichen Verdünnungsmitteln oder Trägern oder mit üblichen Zusätzen, z.B. Farb- oder Geschmacksstoffen, vermischt und als Sirup oder in Form von Kapseln angwewendet werden.

Für die parenterale Verabreichung werden die Liposomen in einer sterilen wässrigen Lösung, welche als Trägerflüssigkeit dient, z.B. steriler, Calcium-freier, isotonischer Kochsalz- oder Glucoselösung, abgepuffert auf pH 7,0—7,8, vorzugsweise 7,2—7,4, dispergiert.

Für die topische Verabreichung wird die auf pH 7,0—7,8, vorzugsweise 7,2—7,4, abgepufferte Liposomen-haltige wässrige Dispersion mit üblichen festen Trägermaterialien, z.B. Verdickern, z.B. Hydroxypropylcellulose, sowie geeigneten Konservierungsmitteln, Antioxydanzien oder Duftstoffen vermischt und als Lotion oder Gel zur Applikation auf der Haut oder auf Schleimhäuten verwendet.

Die Erfindung betrifft im engeren Sinne pharmazeutische Zusammensetzungen enthaltend synthetische, zu mehr als 90 Gew.-% reine Phospholipide in einem Mischungsverhältnis der Phospholipide der Formel I zu Phospholipiden der Formel II von ca. 10 zu 90 bis ca. 50 zu 50 Molprozent und einen pharmazeutischen Wirkstoff oder ein Stoffgemisch aus der Gruppe der Antiphlogistika, Antibiotika, Antileishmanien, Antimykotika, Antineoplastika und Immunomudolatoren und gegebenenfalls eine auf pH 7,0—7,8 abgepufferte Trägerflüssigkeit und/oder zusätzliche feste Trägermaterialien.

Die Erfindung betrifft bevorzugt pharmazeutische Zusammensetzungen enthaltend

a) eine synthetisches, zu mehr als 90 Gew.-% reines Phospholipid der Formel I, worin $R_1$ und $R_2$ gleich sind und 9-cis-Dodecenoyl, 9-cis-Tetradecenoyl, 9-cis-Hexadecenoyl, 9-cis-Octadecenoyl oder 9-cis-Icosenoyl bedeuten, n eins $Y^{\oplus}$ das Natrium ist,

b) ein synthetisches, zu mehr als 90 Gew.-% reines Phospholipid der Formel II, $R_1$ n-Dodecenoyl, n-Tetradecanoyl, n-Hexadecanoyl oder n-Octadecanoyl, $R_2$ 9-cis-Dodecenoyl, 9-cis-Tetradecenoyl, 9-cis-Hexadecenoyl, 9-cis-Octadecenoyl oder 9-cis-Icosenoyl und $R_a$, $R_b$ und $R_c$ Methyl bedeuten,

c) einen pharmazeutischen Wirkstoff oder ein Stoffgemisch aus der Gruppe der Antiphlogistika, Antibiotika, Antileishmanien, Antimykotika, Atineoplastika und Immunmodulatoren und gegebenenfalls eine auf pH 7,2—7,4 abgepufferte Trägerflüssigkeit.

Die Erfindung betrifft insbesondere pharmazeutische Zusammensetzungen enthaltend

a) synthetisches, zu mehr als 90 Gew.-% reines Natrium-1,2-di-(9-cis-octadecenoyl)-3-sn-phosphatidyl-(S)-serin,

b) synthetisches, zu mehr als 90 Gew.-% reines 1-n-Hexadecanoyl-2-(9-cis-octadecenoyl)-3-sn-phosphatidylcholin,

c) einen pharmazeutischen Wirkstoff oder ein Stoffgemisch aus der Gruppe der Antiphlogistika, Antibiotika, Antileishmanien, Antimykotika, Antineoplastika und Immunmodulatoren und gegebenenfalls eine auf pH 7,2—7,4 abgepufferte Trägerflüssigkeit.

Die Erfindung betrifft in erster Linie pharmazeutische Zusammensetzungen enthaltend

a) synthetisches, zu mehr als 90 Gew.-% reines Natrium-1,2-di-(9-cis-octadecenoyl)-3-sn-phosphatidyl-(S)-serin,

b) synthetisches, zu mehr als 90 Gew.-% reines 1-n-Hexadecanoyl-2-(9-cis-octadecenoyl)-3-sn-phosphatidylcholin,

c) einen pharmazeutischen Wirkstoff oder ein Stoffgemisch aus der Gruppe der Antiphlogistika, z.B. Diclofenac oder Pirprofen, Antineoplastika z.B. Mitomycin, Cytarabin, Dactinomycin, Daunorubicin, Doxorubicin oder Etoposid, Immunmodulatoren, z.B. N - Acetyl - D - muramyl - L - alanyl - D - isoglutaminyl - L - alanin - 2 - (1,2 - dipalmitoyl - sn - glycero - 3 - hydroxyphosphoryloxy) - äthylamid, N - Acetyl - D - muramyl - L - alanyl - D - glutaminsäure - ($C_\gamma$) - L - alanin - 2 - (1,2 - dipalmitoyl - sn - glycero - 3 - hydroxyphosphoryloxy) - äthylamid - dinatriumsalz, N - Acetyl - desmethylmuramyl - L - alanyl - D - isoglutaminnatriumsalz, N - Acetyl - D - muramyl - L - alanyl - D - isoglutamin - natriumsalz, N - Acetyl - D - muramyl - L - alanyl - D - glutamin - α - n - butylester, $N^\alpha$ - (N - Acetyl - D - muramyl - L - alanyl - D - isoglutaminyl) - $N^\varepsilon$stearoyl - L - lysin oder 6 - O - Stearoyl - N - acetyl - D - muramyl - L - alanyl - D - isoglutamin, Lymphokine oder Kombinationen davon, und gegebenenfalls eine auf pH 7,2—7,4 abgepufferte Trägerflüssigkeit.

Die Erfindung betrifft hauptsächlich pharmazeutische Zusammensetzungen enthaltend

a) synthetisches, zu mehr als 90 Gew.-% reines Natrium-1,2-di-(9-cis-octadecenoyl)-3-sn-phosphatidyl-(S)-serin,

b) synthetisches, zu mehr als 90 Gew.-% reines 1-n-Hexadecanoyl-2-(9-cis-octadecenoyl)-3-sn-phosphatidylcholin,

c) einen pharmazeutischen Wirkstoff aus der Gruppe Antineoplastika z.B. Mitomycin, Cytarabin, Dactinomycin, Daunorubicin, Doxorubicin oder Etoposid, und Immunmodulatoren, z.B. N - Acetyl - D - muramyl - L - alanyl - D - isoglutaminyl - L - alanin - 2 - (1,2 - dipalmitoyl - sn - glycero - 3 - hydroxyphosphoryloxy) - äthylamid, N - Acetyl - desmethylmuramyl - L - alanyl - D - isoglutamin - natriumsalz, N - Acetyl - D - muramyl - L - alanyl - D - isoglutamin - natriumsalz oder Lymphokine, z.B. humanes Gamma-Inferferon oder Interleuken 2 oder Stoffgemische, welche in Kulturfiltration aus Kulturen mit menschlichen T-Lymphozyten aus Milz oder peripherem Blut nach Stimulierung durch Antigene oder Mitogene oder enthalten und durch hohen Anteil an Makophagenaktivierendem Faktor (MAF) gekennzeichnet sind oder Kombinationen davon, und gegebenenfalls eine auf pH 7,2—7,4 abgepufferte Trägerflüssigkeit.

Die Erfindung betrifft vor allem pharmazeutische Zusammensetzungen enthaltend

a) synthetisches, zu mehr als 90 Gew.-% reines Natrium-1,2-di-(9-cis-octadecenoyl)-3-sn-phosphatidyl-(S)-serin,

b) synthetisches, zu mehr als 90 Gew.-% reines 1-n-Hexadecanoyl-2-(9-cis-octadecenoyl)-3-sn-phosphatidylcholin,

c) einen immunmodulatorisch wirksame Verbindung, z.B. N - Acetyl - D - muramyl - L - alanyl - D - isoglutaminyl - L - alanin - 2 - (1,2 - dipalmitoyl - sn - glycero - 3 - hydroxyphosphoryloxy) - äthylamid, N - Acetyl - desmethylmuramyl - L - alanyl - D - isoglutamin - natriumsalz oder N - Acetyl - D - muramyl - L - alanyl - D - isoglutaminnatriumsalz, gegebenenfalls in Kombination mit Stoffgemischen, welche in Kulturfiltraten aus Kulturen mit menschlichen T-Lymphozyten aus Milz oder peripherem Blut nach Stimulierung durch Antigene oder Mitogene enthalten und durch mindestens 70%-igen Anteil an MAF gekennzeichnet sind, und gegebenenfalls eine auf pH 7,2—7,4 abgepufferte Trägerflüssigkeit.

Die genannten pharmazeutischen Zusammensetzungen können als Trockenpräparate in den Handel gelangen und in Form einer wässrigen Liposomendispersion in einer auf pH 7,0—7,8 abgepufferten Trägerflüssigkeit appliziert werden.

Die Dosismenge für den zu applizierenden Wirkstoff ist im Allgemeinen die für den betreffenden Wirkstoff für die jeweilige Applikationsform, das Alter des Patienten und den gesundheitlichen Zustand des Patienten bekannte und z.B. im Deutsche Arzneimittelbuch (DAB) vorgeschriebene Höchst- und Mindestmenge, Wässrige Dispersionen mit erfindungsgemäss herstellbaren Liposomen haben aber auch den Vorteil, dass Wirkstoffe in geringeren Dosen appliziert zu den Rezeptoren gelangen und dort einen therapeutischen Effekt bewirken können oder bei Applikation von höheren Dosen unerwünschte Nebenwirkungen vermieden werden können.

Die bevorzugte Dosismenge für die genannten, in Form von Liposomen verkapselten Immunmodulatoren des Muramylpeptid- oder Lipopeptid-Typs beträgt 0,01 bis ca. 10 mg/kg Körpergewicht pro Gabe. Für Lymphokine in Liposomenform, z.B. humanes Gamma-Interferon oder

Mischungen enthaltend MAF, beträgt die bevorzugte Dosismenge ca. 100—100,000 Einheiten/ml Gamma-Interferon oder MAF.

Die Erfindung betrifft ebenfalls Mischungen, insbesondere homogene Mischungen aus synthetischen, im wesentlichen reinen Phospholipiden der Formeln I und II, insbesondere Mischungen in einem Mischungsverhältnis der Phospholipide der Formel I zu Phospholipiden der Formel II von ca. 10 zu 90 bis ca. 50 zu 50 Molprozent. Bevorzugt ist das Mischungersverhältnis von 30 zu 70 Molprozent. Diese Mischungen lassen sich zur Herstellung von Liposomen in wässriger Phase enthaltend einen wasserlöslichen Wirkstoff verwenden.

Die erfindungsgemässen pharmazeutischen Zusammensetzungen bzw. die genannten Mischungen werden beispielsweise hergestellt, indem man

a) eine homogene Mischung bestehend aus synthetischen, zu mehr als 90 Gew.-% reinen Phospholipiden der Formeln I und II und einem lipophilen Stoff oder Stoffgemisch bit biologischer Wirkung herstellt und zur Herstellung von Liposomen die erhältliche homogene Mischung in wässriger Phase dispergiert oder

b) eine homogene Mischung bestehend aus synthetischen, zu mehr als 90 Gew.-% reinen Phospholipiden der Formel I und II herstellt und zur Herstellung von Liposomen die erhältliche homogene Mischung in wässriger Phase enthaltend einen wasserlöslichen Stoff oder Stoffgemisch mit biologischer Wirkung dispergiert und,

wenn notwendig, die erhältliche wässrige Dispersion auf pH 7,0—7,8 abpuffert und, wenn erwünscht, die erhältlichen Liposomen anreichert und/oder abtrennt.

Die Herstellung oder homogenen Mischung erfolgt beispielsweise durch Film- oder bevorzugt durch Lyophilisatbildung.

Die Filmbildung erfolgt nach Verfahrensvariante a) durch Auflösen der synthetischen Phospholipide der Formel I und II und des lipophilen, und zu verkapselnden Stoffs oder Stoffgemisches oder nach Verfahrensvariante b) durch Auflösen der synthetischen Phospholipide der Formeln I und II in einem organischen Lösungsmittel mit niedrigem Schmelzpunkt und Entfernen des Lösungsmittels.

Die Auswahl der geeigneten Lösungsmittel zur Herstellung eines Film ist von der Löslichkeit der Lipidkomponenten und der Einschlussverbindungen abhängig. Geeignete Lösungsmittel zur Herstellung der homogen Mischung durch Filmbildung sind beispielsweise niedrig siedende und schmelzende (unterhalb 0°), unsubstituierte oder substituierte, z.B. halogenierte, aliphatische oder cycloaliphatische Kohlenwasserstoffe, z.B. n-Hexan, Cyclohexan, Methylenchlorid oder Chloroform, Alkohole, z.B. Methanol, Niederalkancarbonsäureester, z.B. Essigsäureäthylester, oder Aether, z.B. Diäthyläther, oder Mischungen dieser Lösungsmittel. Man entfernt das Lösungsmittel im Vakuum, vorzugsweise Hochvakuum, oder durch Abblasen mit Intertgas, z.B. Stickstoff.

Die Lyophilisatbildung erfolgt nach Verfahrensvariante a) durch Lyophilisieren einer Lösung der synthetischen Phospholipide der Formeln I und II und des lipophilen zu verkapselnden Stoffs oder Stoffgemisches oder nach Verfahrensvariante b) durch Lyophilisieren einer Lösung der synthetischen Phospholipide der Formeln I und II in einem organischen Lösungsmittel mit hohem Schmelzpunkt auf die in der DE—A—2,818,655 beschrieben Weise. Geeignete Lösungsmittel liegen beim Gefriertrocknen, z.B. bei der Temperatur des Methanol-, Aethanol- oder Aceton-Trockeneisgemisches, zusammen mit den Lipidkomponenten und den Einschlussverbindungen in festem Zustand vor und sind z.B. organische Lösungsmittel mit einem Schmelzpunkt höher als 0°C, z.B. Eisessig, Benzol oder Dioxan, insbesondere tert-Butanol.

Eine homogene Mischung lässt sich auch durch Sprühtrocknung einer Lösung der Phospholipide und der lipophilen Einschlussverbindungen in einem organischen Lösungsmittel, z.B. Chloroform, herstellen. Man erhält die homogene Mischung in Form eines Pulvers.

Zur Herstellung der homogenen Mischung eignet sich ein ungefähres Mischungsverhältnis der Phospholipide der Formel I zu Phospholipiden der Formel II ca. 10 zu 90 bis ca. 50 zu 50 Molprozent, insbesondere 30 zu 70 Molprozent. Das ungefähre Mischungsverhältnis der Einschlussverbindungen zur Gesamtmenge der Lipide beträgt ca. 0,001 bis 1,0 zu 1,0, bevorzugt 0,005 bis 0,1 zu 1,0 Mol.

Man dispergiert beispielsweise durch Schütteln (z.B. Vortex-Mischer) oder Rühren der wässrigen Phase, der man nach Verfahrensvariante a) die zuvor hergestellte homogene Mischung der Phospholipide (I) und (II) und der lipophilen Einschlussverbindungen zugesetzt hat, oder welche nach Verfahrensvariante b) die wasserlösliche Einschlussverbindung enthält und der man die zuvor hergestellte homogene Mischung der Phospholipide (I) und (II) zugesetzt hat. Die Bildung von Liposomen, welche gross, klein, unilamellar oder multilamellar sein können, findet spontan (spontaneous vesiculation), d.h. ohne zusätliche Energiezufuhr von aussen und mit grosser Geschwindigkeit statt. Es können ca. 0,1 bis 50 Gewichtsprozent, vorzugsweise 2 bis 20 Gewichtsprozent (bezogen auf das Gesamtgewicht der wässrigen Dispersion), der homogenen Mischung in wäsriger Phase dispergiert werden.

Man puffert sauer oder basisch reagierende wässrige Dispersionen auf pH 7,0—7,8 vorzugsweise pH 7,2—7,4 ab. Vorzugsweise dispergiert man in einer bereits auf diesen pH-Wert abgepufferten, wässrigen Phase.

Die Verfahrensvariante a) eignet sich besonders, wenn lipophile und schlecht wasserlösliche Wirkstoffe in Form von Liposomen eingeschlossen werden sollen, z.B. Muramyldi- oder Muramyltripeptide. Die Verfahrensvariante b) bietet sich an, wenn eine Liposomenmischung aus

wasserlöslichen Wirkstoffen z.B. Cytarabin oder Zytostatika vom Typ Trifosfamid, hergestellt werden soll.

Die Herstellung der erfindungsgemässen pharmazeutischen Zusammensetzungen in Form von wässrigen Liposomen-Mischungen kann auch nach sämtlichen anderen bisher bekannt gewordenen Verfahren zur Herstellung von Liposomen erfolgen. z.B. auf herkömmliche Weise durch Beschallung mit Ultraschall der wässrigen Dispersion enthaltend die Phospholipide (I) und (II) und die Einschlussverbindungen, durch Infusionsmethoden oder durch Umkehrphasen-Verdampfung.

Man dispergiert bei Temperaturen unterhalb ca. 36°C, vorzugsweise bei Raumtemperatur. Falls es die Empfindlichkeit der zu verkapselnden Verbindungen verlangt, führt man das Verfahren unter Kühlen und gegebenenfalls Inertgasatmosphäre, z.B. Stickstoff- oder Argonatmosphäre, durch. Die erhältlichen Liposomen sind in wässriger Phase sehr lange (bis zu mehreren Wochen oder Monaten) beständig. Pharmazeutische Zusammensetzungen in Form von wässrigen Liposomendispersionen können gegebenenfalls nach Zusatz von Stabilisatoren, z.B. Mannit oder Lactose, durch Gefriertrocknung lagerfähig gemacht werden.

Die Grösse der gebildeten unilamellaren Liposomen ist u.a. von der Struktur des Wirkstoffs und der Lipidkomponenten, dem Mischungsverhältnis der Komponenten und der Konzentration dieser Komponenten in der wässrigen Dispersion abhängig. So kann man beispielsweise durch Erhöhung oder Erniedrigung der Konzentration der Lipidkomponenten wässrige Phasen mit einem hohen Anteil an kleinen oder grossen Liposomen herstellen.

Durch Nachbehandlung der Liposomendispersion, z.B. durch Beschallung mit Ultraschall oder Extrusion durch geradporige Filter (z.B. Nucleopore®) kann man eine besonders einheitliche Grössenverteilung der Liposomen erhalten.

Die Trennung der grossen von den kleinen Liposomen, sofern überhaupt notwendig, erfolgt mittels herkömmlicher Trennmethoden, z.B. Gel-filtration, z.B. mit Sepharose 4B oder Sephacryl als Träger, oder durch Sedimentation der Liposomen in der Ultrazentrifuge z.B. bei 160,000 × g. Beispielsweise setzen sich nach mehrstündigem, z.B. ca. dreistündigem, Zentrifugieren in diesem Schwerefeld grosse Liposomen ab, während die kleinen Liposomen dispergiert bleiben und dekantiert werden können. Nach mehrmaligem Zentrifugieren erreicht man eine vollständige Trennung der grossen von den kleinen Liposomen.

Liposomen werden vorzugsweise abgetrennt, wenn gemäss Verfahrensvariante b) die wässrige Phase nicht verkapselte, wasserlösliche verbindungen enthält. Insbesondere sollten wasserlösliche Antineoplastika, z.B. Alkylanzien, beispielsweise durch Filtration, Ultrafiltration, Dialyse oder Zentrifugieren abgetrennt werden, da die Verträglichkeit dieser Wirkstoffe in gelöster Form nicht besonders gut ist. Die angereicherten Liposomen können mit einer Trägerflüssigkeit, z.B. isotonischer, steriler Kochsalzlösung, vermischt werden. Wässrige Dispersionen mit kleinen Liposomen von relativ einheitlicher Grösse erhält man auch durch Behandlung mit Ultraschall.

Auch durch Gelfiltration kann man alle in der wässrigen Phase befindlichen Liposomen mit einem Durchmessergrösser als $6{,}0 \times 10^{-8}$m sowie nicht verkapselte lipophile Wirkstoffe und überschüssige, dispergierte Lipide, welche in hochmolekularen Aggregaten vorliegen, abtrennen und so eine wässrige Dispersion mit einer Fraktion von Liposomen mit relativ einheitlicher Grösse herstellen.

Die erfolgte Bildung von Liposomen und ihr Gehalt in wässriger Phase lässt sich in an sich bekannter Weise anhand verschiedener physikalischer Messmethoden nachweisen, z.B. mit gefriergebrochenen (freeze fracture) Proben und Dünnschnitten im Elektronemikroskop oder durch Röntgendiffraktion, durch dynamische Lichtstreuung, durch Massenbestimmung des Filtrates in der analytischen Ultrazentrifuge und vor allem spektroskopisch, z.B. im Kernresonanzspektrum ($^{1}$H, $^{13}$C und $^{31}$P).

Synthetische, im wesentlichen reine Phospholipide de Formeln I und II sind bekannt. Beispielsweise ist von Browning J. und Seelig J. im Uebersichtsartikel "Synthesis of Specifically Deuterated Saturated and Unsaturated Phosphatidylserins" in Chem. and Physics of Lipids 24 (1979), 103—118, die Herstellung von 1,2-Di-(9-cis-octadecenoyl)-3-sn-phosphatidyl-(S)-serin beschrieben.

Die pharmazeutischen Wirkstoffe aus der Gruppe der Antiphlogistika, Antibiotika, Antileishmanien und Krebschemotherapeutika sind bekannt, siehe Merck-Index, Tenth Edition oder das weiter vorn erwähnte Uebersichtswerk von Schröder et al. "Pharmazeutische Chemie".

Die genannten Immunmodulatoren vom Muramylpeptid-Typ der Formel VI sind ebenfalls bekannt, siehe beispielsweise Europäische Patentschrift Nr. 25495 und 21367, sowie die Französische Patentschrift Nr. 7,637,091.

Die genannten Immunmodulatoren vom Lipopetid-Typ sind ebenfalls bekannt, z.B. EP—A—114,787 oder Europäische Patentschrift 330.

Die Herstellung von gereinigtem Interleuken 2 ist in EP—A—0106179 sowie in der US-Patentschrift 4,448,879 beschrieben.

Stoffgemische enthaltende Makrophagen-Migration-Inhibitionsfaktor (MIF), Leukozyten-Migration-Inhibitionsfaktor, Leukozyten-Migration-Verstärkungsfaktor, Makrophagen-aktivierender Faktor (MAF), Kolonienstimulierender Faktor etc. sind in zahlreichen Publikationen beschrieben worden, z.B. Salahuddi S.Z. et al. Science 223 (4637) 703—707 (1984). Die Herstellung von Kulturfiltraten enthaltend einen besonders hohen Anteil an humanem MAF ist ebenfalls in zahlreichen Publikationen beschrieben, z.B. Le J. et al. J. Immunology, Vol. 131, 2821—2826 (1983), Kleinermann et al., J. Clinical Investigation, 72, 304—315 (1983), Cameron D. J., J. Clin. Lab. Immunol. (1984), 13, 47—50, sowie Kleinermann E. S. und Fidler I. J., Lymphokine Research, Vol. 2, No. 1, 7—12 (1983).

Die verwendeten Pufferlösungen vom pH 7,0—7,8 sind vorzugsweise sterile Phosphatpufferlösungen auf der Basis Dihydrogen-/ Hydrogenphosphat, welche beispielsweise nach der in Hagers Handbuch der Pharmazeutischen Praxis, Springer Verlag, Band 1 S. 357—359 gegebenen Vorschrift hergestellt werden können. Insbesondere wird sterile, isotonische Calciumfreie Pufferlösung von pH 7.2 (Dulbecco) verwendet.

Die nachfolgenden Beispiele veranschaulichen die Erfindung ohne sie zu beschränken. Temperaturen sind in Graden Celsius angegeben.

Beispiel 1

In einem Rundkolben werden 586 mg steriles tert-Butanol, 0,1 mg N - Acetyl - D - muramyl - L - alanyl - D - isoglutaminyl - L - alanin - 2 - (1,2 - dipalmitoyl - sn - glycero - 3 - hydroxyphosphoryloxy) - äthylamid (Herstellung gemäss Europäische Patentschrift 25 495), 75 mg (95% reines) Natrium - 1,2 - di - (9 - cis - octadecenoyl) - 3 - sn - phospatidyl - (S) - serin (Herstellung gemäss Browning J. und Seelig J., Chem. and Physics of Lipids 24 (1979) 103—118) und 175 mg (95% reines) 1 - n - Hexadecanoyl - 2 - (9 - cis - octadecenoyl) - 3 - sn - phosphatidylcholin (Avanti, Polar Lipids) gelöst. Die Lösung wird über Acrodisc® (2,0 × $10^{-7}$m) sterilfiltriert und nach Abfüllen in eine sterile Viale bei −45° eingefroren. Die Viale wird, bis eine Temperatur von 25° erreicht ist, im Vakuum getrocknet und unter Argonatmosphäre verschlossen.

Vor dem Gebrauch wird zu diesem Trockenpräparat (Lyophilisat), bei Raumtemperatur mit einer sterilen Spritze 2,5 ml sterile, Calcium-freie, Phosphat-gepufferte (pH 7,2—7,4) Kochsalzlösung (Dulbecco) gegeben und die Viale auf einem standardisierten Laborschüttler (Vortex, Stufe 6) zehn Minuten lang geschüttelt. Die entstandene Liposomendispersion ist bei 4° lagerfähig und eignet sich zur parenteralen (i.v.) Applikation.

Beispiel 2

In einem Rundkolben werden 586 mg steriles tert-Butanol, 0,1 mg N - Acetyl - D - muramyl - L - alanyl - D - isoglutaminyl - L - alanin - 2 - (1,2 - dipalmitoyl - sn - glycero - 3 - hydroxyphosphoryloxy) - äthylamid (Herstellung gemäss Europäische Patentschrift 25 495), 75 mg (95% reines) Natrium - 1,2 - di - (9 - cis - octadecenoyl) - 3 - sn - phospatidyl - (S) - serin (Herstellung gemäss Browning J. und Seelig J., Chem. and Physics of Lipids 24 (1979) 103—118) und 175 mg (95% reines) 1 - n - Hexadecanoyl - 2 - (9 - cis - octadecenoyl) - 3 - sn - phosphatidylcholin (Avanti, Polar Lipids) gelöst. Die Lösung wird über Acrodisc® (2,0 × $10^{-7}$m) sterilfiltriert und in eine sterile Viale gefüllt. Die Viale wird bei 150 Upm zum Rotieren gebracht und das Lösungsmittel in einem Strom von gereinigtem, bei 1 bar filtriertem Stickstoff abgeblasen. Anschliessend wird die Viale im Hochvakuum von 6,0 × $10^{-2}$ mbar evakuiert. Die Viale wird unter Argon-Schutzgasatmosphäre verschlossen.

Vor Gebrauch wird zu diesem hergestellt en Film bei Raumtemperatur mit einer sterilen Spritze 2,5 ml sterile, Calcium-freie, Phosphat-gepufferte (pH 7,2—7,4) Kochsalzlösung (Dulbecco) gegeben und die Viale auf einem standardisierten Laborschüttler (Vortex, Stufe 6) zehn Minuten lang geschüttelt. Die entstandene Liposomendispersion ist bei 4° lagerfähig und eignet sich zur parenteralen (i.v.) Applikation.

Beispiel 3

Analog Beispiel 2 lassen sich wässrige Dispersionen enthaltend Liposomen bestehend aus 75 mg (0,091 mMol) Natrium - 1,2 - di - (9 - cis - octadecenoyl) - 3 - sn - phospatidylserin, 175 mg (0,231 mMol) 1 - n - Hexadecanoyl - 2 - (9 - cis - octadecenoyl) - 3 - sn - - phosphatidylcholin und mehr als 0,1 mg bis 10 mg N - Acetyl - D - muramyl - L - alanyl - D - isoglutaminyl - L - alanin - 2 - (1,2 - dipalmitoyl - sn - glycero - 3 - hydroxyphosphoryloxy) - äthylamid herstellen.

Beispiel 4

In einem Rundkolben werden 586 mg steriles tert-Butanol, 75 mg (95% reines) Natrium - 1,2 - di - (9 - cis - octadecenoyl) - 3 - sn - phosphatidyl - (S) - serin und 175 mg 95% reines) 1 - n - Hexadecanoyl - 2 - (9 - cis - octadecenoyl) - 3 - sn - phosphatidylcholin gelöst, über Acrodisc® (2,0 × $10^{-7}$m) sterilfiltriert und in eine sterile Viale gefüllt. Die Viale wird bei 1750 Upm zum Rotieren gebracht und das Lösungsmittel in einem Strom von gereinigtem, bei 1 bar filtriertem Stickstoff abgeblasen. Anschliessend wird die Viale im Hochvakuum von 6,0 × $10^{-2}$ mbar evakuiert. Die Viale wird unter Argon-Schutzgasatmosphäre verschlossen.

Vor Gebrauch wird zu diesem hergestellten Film bei Raumtemperatur mit einer sterilen Spritze 2,5 ml sterile, Calcium-freie, Phosphat-gepufferte (pH 7,2—7,4) Kochsalzlösung (Dulbecco) enthaltend Doxorubicin in einer Konzentration von 4 g/l gegeben und die Viale auf einem standardisierten Laborschüttler (Vortex, Stufe 6) zehn Minuten lang geschüttelt. Anschliessend zentrifugiert man die Dispersion bei 40000 g sechzig Minuten lang. Man entfernt das Filtrat und suspendiert die Liposomen in 2,5 ml 0,85%-iger, steriler Kochsalzlösung.

Beispiel 5

In einer Viale (15 ml) werden 506 mg steriles tert-Butanol, 75 mg (98% reines) Natrium - 1,2 - di - (9 - cis - octadecenoyl) - 3 - sn - phosphatidyl - (S) - serin (Herstellung gemäss Browning J. und Seelig J.,

Chem. and Physics of Lipids 24 (1979) 103) und 175 mg (99,5% reines) 1 - n - Hexadecanoyl - 2 - (9 - cis - octadecenoyl) - 3 - sn - - phosphatidylcholin (Herstellung gemäss Eibl H., Chem. Physics of Lipids 26 (1980) 239 und Eibl H., Angewandte Chemie 96 (1984) 247) gelöst. Die Lösung wird über Acrodisc® (2,0 × $10^{-7}$m) sterilfiltriert und nach Abfüllen in einer sterilen Viale bei −45° eingefroren. Die Viale wird, bis man eine Temperatur von 25° erreicht, im Vakuum getrocknet und unter Argonatmosphäre verschlossen.

Vor Gebrauch wird zu diesem Trockenpräparat mit einer sterilen Spritze 2,5 ml sterile, Calcium-freie, Phosphat-gepufferte Kochsalzlösung (Dulbecco) enthaltend Doxorubicin in einer Konzentration von 4 g/l gegeben und die Viale auf einem standardisierten Laborschüttler (Vortex, Stufe 6) eine Minute lang geschüttelt. Nach Zugabe von 22,5 ml steriler, Calcium-freier, Phosphat-gepufferter Kochsalzlösung, equilibriert bei 10°, wird die Suspension 2 Minuten lang geschüttelt und anschliessend bei 10,000 g und 10°, dreissig Minuten lang zentrifugiert. Man entfernt den Rückstand und resuspendiert die Liposomen in 25 ml der sterilen Kochsalzlösung bei 10°. Man wiederholt das Zentrifugationsverfahren, nachdem man die Liposomen in 2,5 ml 0,85%-iger steriler Kochsalzlösung resuspendiert hat. Die Liposomensuspension enthält 9 mg Doxorubicin per 250 mg Lipid und ist geeignet zur i.v. Applikation.

Beispiel 6

Analog Beispiel 5 lassen sich Doxorubicin enthaltende Liposomen aus einem Trockenpräparat enthaltend 225 mg (0,297 mMol) (99,5% reines) 1 - n - Hexadecanoyl - 2 - (9 - cis - octadecenoyl) - 3 - sn - phosphatidylcholin und 25 mg (0,030 mMol) (98% reines) Natrium - 1,2 - di - (9 - cis - octadecenoyl) - 3 - sn - phosphatidyl - (S) - serin herstellen. Die Liposomensuspension enthält 7 mg Doxorubicin pro 250 mg Lipid und ist geeignet zur i.v. Applikation.

Beispiel 7

Analog den Beispielen 4 und 5 lässt sich eine Liposomendispersion herstellen und zwar durch Dispersion eines Trockenpräparates der Phospholipide in 2,5 ml steriler, Calcium-freier, Phosphat-gepufferter (pH 7,2—7,4) Kochsalzlösung (Dulbecco) enthaltend Mitomycin C in einer Konzentration von 1 g/l, Zentrifugieren und Suspendieren in einer Kochsalzlösung.

Beispiel 8

Analog Beispiel 5, aber unter Auslassung der Zentrifugation, lässt sich eine Liposomendispersion herstellen und zwar durch Dispersion eines Trockenpräparates der Phospholipide in 2,5 ml steriler, Calcium-freier, Phosphat-gepufferter (pH 7,2—7,4) Kochsalzlösung (Dulbecco) enthaltend fünzig bis zweihundert Mikrogramm Natrium - N - acetyl - D - muramyl - L - alanyl - D - isoglutamine (British Patent Specification 1,570,625) und tausend bis zehntausend Einheiten rekombiniertes, menschliches Gamma-Interferon isoliert gemäss Patent Application 121.157 (Kyowa Hakko Kogyo Co.).

Beispiel 9

Analog Beispiel 5, aber unter Auslassung der Zentrifugation, lässt sich eine Liposomendispersion herstellen und zwar durch Dispersion eines Trockenpräparates der Phospholipide in 2,5 ml steriler, Calcium-freier, Phosphat-gepufferter (pH 7,2—7,4) Kochsalzlösung (Dulbecco) enthaltend fünfzig bis zweihundert Mikrogrammen Natrium - N - acetyldesmethyl - muramyl - L - alanyl - D - isoglutamine und tausend bis hunderttausend Einheiten rekombiniertes humanes Gamma-Interferon.

Beispiel 10

Analog Beispiel 4, aber unter Auslassung des Zentrifugationsverfahrens, lässt sich eine Liposomensuspension herstellen und zwar durch Dispersion eines Trockenpräparates der Phospholipide in 2,5 ml steriler, Calcium-freier, Phosphat-gepufferter (pH 7,2—7,4) Kochsalzlösung (Dulbecco) enthaltend fünfzig bis zweihundert Mikrogramm Natrium - N - acetyl - D - muramyl - L - alanyl - D - isoglutamine und tausend bis hundert-tausend Einheiten rekombiniertes, humanes Gamma-Interferon.

Beispiel 11

Analog Beispiel 4, aber unter Auslassung der Zentrifugation, lässt sich eine Liposomensuspension herstellen und zwar durch Dispersion eines Trockenpräparates der Phospholipide in 2,5 ml steriler, Calcium-freier, Phosphat-gepufferter (pH 7,2—7,4) Kochsalzlösung (Dulbecco) enthaltend circa fünfzig bis zweihundert Mikrogramm Natrium - N - acetyldesmethylmuramyl - L - alanyl - D - isoglutamine und tausend bis hundert-tausend Einheiten rekombiniertes, humanes Gamma-Interferon.

Beispiel 12

In einem Rundkolben, worin sich 1 g Sorbitol (Kristallgrösse 125—500 μm) befindet, werden tropfenweise, unter Vakuum, 14,2 ml steriles tert-Butanol enthaltend 1 bis 10 mg N - acetyl - D - muramyl - L - alanyl - D - isoglutaminyl - L - alanyl - 2 - (1,2 - dipalmitoyl - sn - glycero - 3 - hydroxyphosphoryloxy) - äthylamid (Herstellung gemäss Europäische Patentschrifft 25 495), 75 mg Natrium - 1,2 - di - (9 - cis - octadecenoyl) - 3 - sn - phosphatidyl - (S) - serin und 175 mg 1 - n - Hexadecanoyl - 2 - (9 - cis - octadecenoyl) - sn - phosphatidylcholin zugefügt. Anschliessend wird das Lösungsmittel

unter Vakuum mit kontrollierten Temperaturbedingungen entfernt. Das entstandene Trockenpulverpräparat wird unter Argonatmosphäre verschlossen.

Vor dem Gebrauch wird zu diesem Trockenpräparat bei Raumtemperatur mit einer sterilin Spritze 20 ml destilliertes steriles Wasser gegeben und der Kolben zehn Minuten lang geschüttelt.

Beispiel 13

Analog Beispiel 12 kann man anstatt Sorbitol durch Glucose, Sucrose, Lactose oder Natriumchlorid als Trägermaterial verwenden.

**Patentansprüche für die Vertragsstaaten: BE CH DE FR GB IT LI LU NL SE**

1. Pharmazeutische Zusammensetzungen enthaltend

a) ein synthetisches, zu mehr als 90 Gew.-% reines Phospholipid der Formel

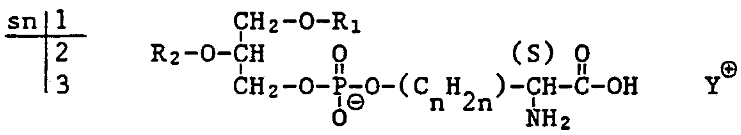

(I),

worin $R_1$ und $R_2$ unabhängig voneinander $C_{10}$—$C_{20}$-Alkenoyl mit gerader Anzahl an C-Atomen, n eine ganze Zahl von eins bis drei und $Y^{\oplus}$ das Kation einer pharmazeutischen annehmbaren Base darstellen,

b) ein synthetisches, zu mehr als 90% Gew.-% reines Phospholipid der Formel

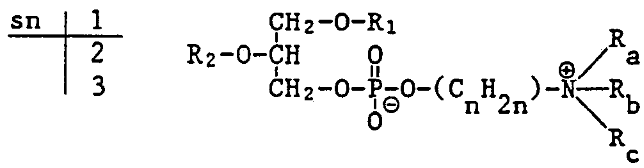

(II),

worin $R_1$ $C_{10}$—$C_{20}$-Alkanoyl mit gerader Anzahl an C-Atomen, $R_2$ $C_{10}$—$C_{20}$-Alkenoyl mit gerader Anzahl an C-Atomen, $R_a$, $R_b$ und $R_c$ Wasserstoff oder $C_1$—$C_4$-Alkyl und n eine ganze Zahl von zwei bis vier darstellen,

c) einen Stoff oder ein Stoffgemisch mit biologischer Wirkung und gegebenenfalls eine Trägerflüsigkeit und/oder zusätzliche feste Trägermaterialien.

2. Pharmazeutische Zusammensetzungen gemäss Anspruch 1 enthaltend synthetische, zu mehr als 90 Gew.-% reine Phospholipid in einem Mischungsverhältnis der Phospholipide der Formel I zu Phospholipiden der Formel II von ca. 10 zu 90 bis ca. 50 zu 50 Molprozent und einen pharmazeutischen Wirkstoff oder ein pharmazeutisches Wirkstoffgemisch und gegebenenfalls eine auf pH 7,0—7,8 abgepufferte Trägerflüssigkeit und/oder zusätzliche feste Trägermaterialien.

3. Pharmazeutische Zusammensetzungen gemäss Anspruch 1 oder 2 enthaltend

a) eine synthetisches, zu mehr als 90 Gew.-% reines Phospholipid der Formel I, worin $R_1$ und $R_2$ gleich sind und 9-cis-Dodecenoyl, 9-cis-Tetradecenoyl, 9-cis-Hexadecenoyl, 9-cis-Octadecenoyl oder 9-cis-Icosenoyl bedeuten, n eins $Y^{\oplus}$ das Natriumion ist,

b) ein synthetisches, zu mehr als 90 Gew.-% reines Phospholipid der Formel II, worin $R_1$ n-Dodecenoyl, n-Tetradecanoyl, n-Hexadecanoyl oder n-Octadecanoyl, $R_2$ 9-cis-Dodecenoyl, 9-cis-Tetradecenoyl, 9-cis-Hexadecenoyl, 9-cis-Octadecenoyl oder 9-cis-Icosenoyl und $R_a$, $R_b$ und $R_c$ Methyl bedeuten,

c) einen pharmazeutischen Wirkstoff oder ein pharmazeutisches Wirkstoffgemisch und gegebenenfalls eine auf pH 7,2—7,4 abgepufferte Trägerflüssigkeit.

4. Pharmazeutische Zusammensetzungen gemäss Anspruch 3 enthaltend

a) synthetisches, zu mehr als 90 Gew.-% reines Natrium-1,2-di-(9-cis-octadecenoyl)-3-sn-phosphatidyl-(S)-serin,

b) synthetisches, zu mehr als 90 Gew.-% reines 1-n-Hexadecanoyl-2-(9-cis-octadecenoyl)-3-sn-phosphatidylcholin,

c) einen pharmazeutischen Wirkstoff oder ein pharmazeutisches Wirkstoffgemisch und gegebenenfalls eine auf pH 7,2—7,4 abgepufferte Trägerflüssigkeit.

5. Pharmazeutische Zusammensetzungen gemäss Anspruch 1 enthaltend synthetische, zu mehr als 90 Gew.-% reine Phospholipide in einem Mischungsverhältnis der Phospholipide der Formel I zu Phospholipiden der Formel II von ca. 10 zu 90 bis ca. 50 zu 50 Molprozent und einen pharmazeutischen Wirkstoff oder ein Stoffgemisch aus der Gruppe der Antiphlogistika, Antibiotika, Antileishmanien, Antimykotika, Antineoplastika and Immunmodulatoren und gegebenenfalls eine auf pH 7,0—7,8 abgepufferte Trägerflüssigkeit und/oder zusätzliche feste Trägermaterialien.

6. Pharmazeutische Zusammensetzungen gemäss Anspruch 1 oder 2 enthaltend

a) eine synthetisches, zu mehr als 90 Gew.-% reines Phospholipid der Formel I, worin $R_1$ und $R_2$ gleich

sind und 9-cis-Dodecenoyl, 9-cis-Tetradecenoyl, 9-cis-Hexadecenoyl, 9-cis-Octadecenoyl oder 9-cis-Icosenoyl bedeuten, n eins und $Y^{\oplus}$ das Natriumion ist,

b) ein synthetisches, zu mehr als 90 Gew.-% reines Phospholipid der Formel II, worin $R_1$ n-Dodecanoyl, n-Tetradecanoyl, n-Hexadecanoyl oder n-Octadecanoyl, $R_2$ 9-cis-Dodecenoyl, 9-cis-Tetradecenoyl, 9-cis-Hexadecenoyl, 9-cis-Octadecenoyl oder 9-cis-Icosenoyl und $R_a$, $R_b$ und $R_c$ Methyl bedeuten,

c) einen pharmazeutischen Wirkstoff oder ein Stoffgemisch aus der Gruppe der Antiphlogistika, Antibiotika, Antileishmanien, Antimykotika, Antineoplastika und Immunmodulatoren und gegebenenfalls eine auf pH 7,2—7,4 abgepufferte Trägerflüssigkeit.

7. Pharmazeutische Zusammensetzungen gemäss Anspruch 3 enthaltend

a) synthetisches, zu mehr als 90 Gew.-% reines Natrium-1,2-di-(9-cis-octadecenoyl)-3-sn-phosphatidyl-(S)-serin,

b) synthetisches, zu mehr als 90 Gew.-% reines 1-n-Hexadecanoyl-2-(9-cis-octadecenoyl)-3-sn-phosphatidylcholin,

c) einen pharmazeutischen Wirkstoff oder ein Stoffgemisch aus der Gruppe der Antiphlogistika, Antibiotika, Antileishmanien, Antimykotika, Antineoplastika und Immunmodulatoren und gegebenenfalls eine auf pH 7,2—7,4 abgepufferte Trägerflüssigkeit.

8. Pharmazeutische Zusammensetzungen gemäss Anspruch 4 enthaltend

a) synthetisches, zu mehr als 90 Gew.-% reines Natrium-1,2-di-(9-cis-octadecenoyl)-3-sn-phosphatidyl-(S)-serin,

b) synthetisches, zu mehr als 90 Gew.-% reines 1-n-Hexadecanoyl-2-(9-cis-octadecenoyl)-3-sn-phosphatidylcholin,

c) einen pharmazeutischen Wirkstoff oder ein Stoffgemisch aus der Gruppe Diclofenac, Pirprofen, Mitomycin, Cytarabin, Dactinomycin, Daunorubicin, Doxorubicin, Etoposid, N - Acetyl - D - muramyl - L - alanyl - D - isoglutaminyl - L - alanin - 2 - (1,2 - dipalmitoyl - sn - glycero - 3 - hydroxyphosphoryloxy) - äthylamid, N - Acetyl - D - muramyl - L - alanyl - D - glutaminsäure - ($C_\gamma$) - L - alanin - 2 - (1,2 - dipalmitoyl - sn - glycero - 3 - hydroxyphosphoryloxy) - äthylamid - dinatriumsalz, N - Acetyl - desmethylmuramyl - L - alanyl - D - isoglutamin - natriumsalz, N - Acetyl - D - muramyl - L - alanyl - D - isoglutamin - natriumsalz, N - Acetyl - D - muramyl - L - alanyl - D - glutamin - α - n - butylester, $N^\alpha$ - (N - Acetyl - D - muramyl - L - alanyl - D - isoglutaminyl) - $N^\varepsilon$stearoyl - L - lysin oder 6 - O - Stearoyl - N - acetyl - D - muramyl - L - alanin - D - isoglutamin, Lymphokine oder Kombinationen davon, und gegebenenfalls eine auf pH 7,2—7,4 abgepufferte Trägerflüssigkeit.

9. Pharmazeutische Zusammensetzungen gemäss Anspruch 4 enthaltend

a) synthetisches, zu mehr als 90 Gew.-% reines Natrium-1,2-di-(9-cis-octadecenoyl)-3-sn-phosphatidyl-(S)-serin,

b) synthetisches, zu mehr als 90 Gew.-% reines 1-n-Hexadecanoyl-2-(9-cis-octadecenoyl)-3-sn-phosphatidylcholin,

c) einen pharmazeutischen Wirkstoff aus der Gruppe Mitomycin, Cytarabin, Dactinomycin, Daunorubicin, Doxorubicin, Etoposid, N - Acetyl - D - muramyl - L - alanyl - D - isoglutaminyl - L - alanin - 2 - (1,2 - dipalmitoyl - sn - glycero - 3 - hydroxyphosphoryloxy) - äthylamid, N - Acetyl - desmethylmuramyl - L - alanyl - D - isoglutamin - natriumsalz oder N - Acetyl - D - muramyl - L - alanyl - D - isoglutamin - natriumsalz, gegebenenfalls in Kombination mit humanem Gamma-Interferon oder Interleukin 2 oder Stoffgemischen, welche in Kulturfiltraten aus Kulturen mit menschlichen T-Lymphozyten aus Milz oder peripherem Blut nach Stimulierung durch Antigene oder Mitogene enthalten und durch hohen Anteil an Makrophagen-aktivierendem Faktor (MAF) gekennzeichnet sind, und gegebenenfalls eine auf pH 7,2—7,4 abgepufferte Trägerflüssigkeit.

10. Pharmazeutische Zusammensetzungen gemäss Anspruch 4 enthaltend

a) synthetisches, zu mehr als 90 Gew.-% reines Natrium-1,2-di-(9-cis-octadecenoyl)-3-sn-phosphatidyl-(S)-serin,

b) synthetisches, zu mehr als 90 Gew.-% reines 1-n-Hexadecanoyl-2-(9-cis-octadecenoyl)-3-sn-phosphatidylcholin,

c) N - Acetyl - D - muramyl - L - alanyl - D - isoglutaminyl - L - alanin - 2 - (1,2 - dipalmitoyl - sn - glycero - 3 - hydroxyphosphoryloxy) - äthylamid, N - Acetyl - desmethylmuramyl - L - alanyl - D - isoglutamin - natriumsalz oder N - Acetyl - D - muramyl - L - alanyl - D - isoglutaminnatriumsalz, gegebenenfalls in Kombination mit Stoffgemischen, welche in Kulturfiltraten aus Kulturen mit menschlichen T-Lymphozyten aus Milz oder peripherem Blut nach Stimulierung durch Antigene oder Mitogene enthalten und durch mindestens 70%-igen Anteil an MAF gekennzeichnet sind, und gegebenenfalls eine auf pH 7,2—7,4 abgepufferte Trägerflüssigkeit.

11. Pharmazeutische Zusammensetzungen gemäss Anspruch 4 enthaltend

a) synthetisches, zu mehr als 90 Gew.-% reines Natrium-1,2-di-(9-cis-octadecenoyl)-3-sn-phosphatidyl-(S)-serin,

b) synthetisches, zu mehr als 90 Gew.-% reines 1-n-Hexadecanoyl-2-(9-cis-octadecenoyl)-3-sn-phosphatidylcholin,

c) N - Acetyl - D - muramyl - L - alanyl - D - isoglutaminyl - L - alanin - 2 - (1,2 - dipalmitoyl - sn - glycero - 3 - hydroxyphosphoryloxy) - äthylamid, und gegebenenfalls eine auf pH 7,2—7,4 abgepufferte Trägerflüssigkeit.

12. Pharmazeutische Zusammensetzungen gemäss Anspruch 4 enthaltend

a) synthetisches, zu mehr als 90 Gew.-% reines Natrium-1,2-di-(9-cis-octadecenoyl)-3-sn-phosphatidyl-(S)-serin,

b) synthetisches, zu mehr als 90 Gew.-% reines 1-n-Hexadecanoyl-2-(9-cis-octadecenoyl)-3-sn-phosphatidylcholin,

c) natürliches oder rekombiniertes, humanes Gamma-Interferon und gegebenenfalls eine auf pH 7,2—7,4 abgepufferte Trägerflüssigkeit.

13. Pharmazeutische Zusammensetzungen gemäss Anspruch 9 enthaltend

a) synthetisches, zu mehr als 90 Gew.-% reines Natrium-1,2-di-(9-cis-octadecenoyl)-3-sn-phosphatidyl-(S)-serin,

b) synthetisches, zu mehr als 90 Gew.-% reines 1-n-Hexadecanoyl-2-(9-cis-octadecenoyl)-3-sn-phosphatidylcholin,

c) rekombiniertes, humanes Gamma-Interferon mit folgender Aminosäure-sequenz:

$H_2N$-Cys-Tyr-Cys-Gln-Asp-Pro-Tyr-Val-Gln-Glu-Ala-Glu-Asn-Leu-Lys-Lys-
Tyr-Phe-Asn-Ala-Gly-His-Ser-Asp-Val-Ala-Asp-Asn-Gly-Thr-Leu-Phe-Leu-
Gly-Ile-Leu-Lys-Asn-Trp-Lys-Glu-Glu-Ser-Asp-Arg-Lys-Ile-Met-Gln-Ser-
Gln-Ile-Val-Ser-Phe-Tyr-Phe-Lys-Leu-Phe-Lys-Asn-Phe-Lys-Asp-Asp-Gln-
Ser-Ile-Gln-Lys-Ser-Val-Glu-Thr-Ile-Lys-Glu-Asp-Met-Asn-Val-Lys-Phe-
Phe-Asn-Ser-Asn-Lys-Lys-Lys-Arg-Asp-Asp-Phe-Glu-Lys-Leu-Thr-Asn-Tyr-
Ser-Val-Thr-Asp-Leu-Asn-Val-Gln-Arg-Lys-Ala-Ile-His-Glu-Leu-Ile-Gln-
Val-Met-Ala-Glu-Leu-Ser-Pro-Ala-Ala-Lys-Thr-Glu-Lys-Arg-Lys-Arg-Ser-
Gln-Met-Leu-Phe-Gln-Gly-Arg-Arg-Ala-Ser-Gln-OH

und gegebenenfalls eine auf pH 7,2—7,4 abgepufferte Trägerflüssigkeit.

14. Pharmazeutische Zusammensetzungen gemäss Anspruch 9 enthaltend

a) synthetisches, zu mehr als 90 Gew.-% reines Natrium-1,2-di-(9-cis-octadecenoyl)-3-sn-phosphatidyl-(S)-serin,

b) synthetisches, zu mehr als 90 Gew.-% reines 1-n-Hexadecanoyl-2-(9-cis-octadecenoyl)-3-sn-phosphatidylcholin,

c) rekombiniertes, humanes Gamma-Interferon mit folgenden Aminosäure-sequenz:

$H_2N$-Cys-Tyr-Cys-Gln-Asp-Pro-Tyr-Val-Lys-Glu-Ala-
Glu-Asn-Leu-Lys-Lys-Tyr-Phe-Asn-Ala-Gly-His-Ser-
Asp-Val-Ala-Asp-Asn-Gly-Thr-Leu-Phe-Leu-Gly-Ile-
Leu-Lys-Asn-Trp-Lys-Glu-Glu-Ser-Asp-Arg-Lys-Ile-
Met-Gln-Ser-Gln-Ile-Val-Ser-Phe-Tyr-Phe-Lys-Leu-
Phe-Lys-Asn-Phe-Lys-Asp-Asp-Gln-Ser-Ile-Gln-Lys-
Ser-Val-Glu-Thr-Ile-Lys-Glu-Asp-Met-Asn-Val-Lys-
Phe-Phe-Asn-Ser-Asn-Lys-Lys-Lys-Arg-Asp-Asp-Phe-
Glu-Lys-Leu-Thr-Asn-Tyr-Ser-Val-Thr-Asp-Leu-Asn-
Val-Gln-Arg-Lys-Ala-Ile-His-Glu-Leu-Ile-Gln-Val-
Met-Ala-Glu-Leu-Ser-Pro-Ala-Ala-Lys-Thr-Gly-Lys-
Arg-Lys-Arg-Ser-Gln-Met-Leu-Phe-Arg-Gly-Arg-Arg-
Ala-Ser-Gln-OH

und gegebenenfalls eine auf pH 7,2—7,4 abgepufferte Trägerflüssigkeit.

15. Mischungen geeignet zur Herstellung von Liposomen bestehend aus synthetischen, zu mehr als 90 Gew.-% reinen Phospholipiden der Formeln I und II gemäss Anspruch 1.

16. Verfahren zur Herstellung von pharmazeutischen Zusammensetzungen gemäss einem der Ansprüche 1—11 dadurch gekennzeichnet, dass man

a) eine homogene Mischung bestehend aus synthetischen, zu mehr als 90 Gew.-% reinen Phospholipiden der Formeln I und II une einem lipophilen Stoff oder einem Stoffgemisch mit biologischer Wirkung herstellt und zur Herstellung von Liposomen die erhältliche homogene Mischung in wässriger Phase dispergiert oder

b) eine homogene Mischung bestehend aus synthetischen,. zu mehr als 90 Gew.-% reinen Phospholipiden der Formeln I und II herstellt und zur Herstellung von Liposomen die erhältliche homogene Mischung in wässriger Phase enthaltend einen wässerlöslichen Stoff oder ein Stoffgemisch mit biologischer Wirkung dispergiert und, wenn notwendig, die erhältliche wässrige Dispersion auf pH 7,0—7,8 abpuffert und, wenn erwünscht, die erhältlichen Liposomen anreichert und/oder abtrennt.

17. Pharmazeutische Zusammensetzung gemäss Anspruch 1 zur Anwendung bei der Behandlung des menschlichen oder tierischen Körpers.

**Patentansprüche für den Vertragsstaat: AT**

1. Verfahren zur Herstellung von pharmazeutischen Zusammensetzungen enthaltend

a) ein synthetisches, zu mehr als 90 Gew.-% reines Phospholipid der Formel

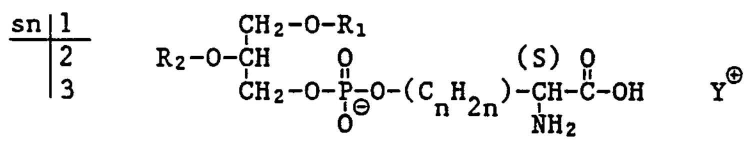 (I),

worin $R_1$ und $R_2$ unabhängig voneinander $C_{10}$—$C_{20}$-Alkenoyl mit gerader Anzahl an C-Atomen, n eine ganze Zahl von eins bis drei und $Y^{\oplus}$ das Kation einer pharmazeutisch annehmbaren Base darstellen,

b) ein synthetisches, zu mehr als 90 Gew.-% reines Phospholipid der Formel

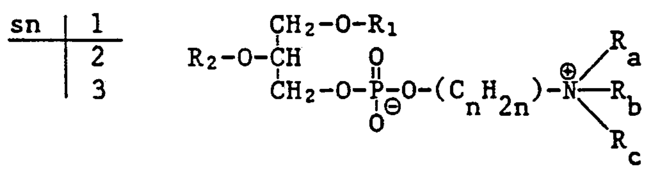 (II) ,

worin $R_1$ $C_{10}$—$C_{20}$-Alkanoyl mit gerader Anzahl an C-Atomen, $R_2$ $C_{10}$—$C_{20}$-Alkenoyl mit gerader Anzahl an C-Atomen, $R_a$, $R_b$ und $R_c$ Wasserstoff oder $C_1$—$C_4$-Alkyl und n eine ganze Zahl von zwei bis vier darstellen,

c) einen Stoff oder ein Stoffgemisch mit biologischer Wirkung und gegebenenfalls eine Trägerflüsigkeit und/oder zusätzliche feste Trägermaterialien, dadurch gekennzeichnet, dass man

a) eine homogene Mischung bestehend aus synthetischen, zu mehr als 90 Gew.-% reinen Phospholipiden der Formeln I und II und einem lipophilen Stoff oder einen Stoffgemisch mit biologischer Wirkung herstellt und zur Herstellung von Liposomen die erhältliche homogene Mischung in wässriger Phase dispergiert oder

b) eine homogene Mischung bestehend aus synthetischen, zu mehr als 90 Gew.-% reinen Phospholipiden der Formeln I und II herstellt und zur Herstellung von Liposomen die erhältliche homogene Mischung in wässriger Phase enthaltend einen wasserlöslichen Stoff oder ein Stoffgemisch mit biologischer Wirkung dispergiert und, wenn notwendig, die erhältliche wässrige Dispersion auf pH 7,0—7,8 abpuffert und, wenn erwünscht, die erhältlichen Liposomen anreichert und/oder abtrennt.

2. Verfahren zur Herstellung von pharmazeutischen Zusammensetzungen gemäss Anspruch 1 enthaltend synthetische, zu mehr als 90 Gew.-% reine Phospholipid in einem Mischungsverhältnis der Phospholipide der Formel I zu Phospholipiden der Formel II von ca. 10 zu 90 bis ca. 50 zu 50 Molprozent und einen pharmazeutischen Wirkstoff oder ein pharmazeutisches Wirkstoffgemisch und gegebenenfalls eine auf pH 7,0—7,8 abgepufferte Trägerflüssigkeit und/oder zusätzliche feste Trägermaterialien, dadurch gekennzeichnet, dass man die in Anspruch 1 genannten Massnahmen ausführt.

3. Verfahren zur Herstellung von pharmazeutischen Zusammensetzungen gemäss Anspruch 1 oder 2 enthaltend

a) eine synthetisches, zu mehr als 90 Gew.-% reines Phospholipid der Formel I, worin $R_1$ und $R_2$ gleich sind und 9-cis-Dodecenoyl, 9-cis-Tetradecenoyl, 9-cis-Hexadecenoyl, 9-cis-Octadecenoyl oder 9-cis-Icosenoyl bedeuten, n eins und $Y^{\oplus}$ das Natriumion ist,

b) ein synthetisches, zu mehr als 90 Gew.-% reines Phospholipid der Formel II, worin $R_1$ n-Dodecanoyl, n-Tetradecanoyl, n-Hexadecanoyl oder n-Octadecanoyl, $R_2$ 9-cis-Dodecenoyl, 9-cis-Tetradecenoyl, 9-cis-Hexadecenoyl, 9-cis-Octadecenoyl oder 9-cis-Icosenoyl und $R_a$, $R_b$ und $R_c$ Methyl bedeuten,

c) einen pharmazeutischen Wirkstoff oder ein pharmazeutisches Wirkstoffgemisch und gegebenenfalls

eine auf pH 7,2—7,4 abgepufferte Trägerflüssigkeit, dadurch gekennzeichnet, dass man die in Anspruch 1 genannten Massnahmen ausführt.

4. Verfahren zur Herstellung von pharmazeutischen Zusammensetzungen gemäass Anspruch 3 enthaltend

a) synthetisches, zu mehr als 90 Gew.-% reines Natrium-1,2-di-(9-cis-octadecenoyl)-3-sn-phosphatidyl-(S)-serin,

b) synthetisches, zu mehr als 90 Gew.-% reines 1-n-Hexadecanoyl-2-(9-cis-octadecenoyl)-3-sn-phosphatidylcholin,

c) einen pharmazeutischen Wirkstoff oder ein pharmazeutisches Wirkstoffgemisch und gegebenenfalls eine auf pH 7,2—7,4 abgepufferte Trägerflüssigkeit, dadurch gekennzeichnet, dass man die in Anspruch 1 genannten Massnahmen ausführt.

5. Verfahren zur Herstellung von pharmazeutischen Zusammensetzungen gemäss Anspruch 1 enthaltend synthetische, zu mehr als 90 Gew.-% reine Phospholipide in einem Mischungsverhältnis der Phospholipide der Formel I zu Phospholipiden der Formel II von ca. 10 zu 90 bis ca. 50 zu 50 Molprozent und einen pharmazeutischen Wirkstoff oder ein Stoffgemisch aus der Gruppe der Antiphlogistika, Antibiotika, Antileishmanien, Antimykotika, Antineoplastika and Immunmodulatoren und gegebenenfalls eine auf pH 7,0—7,8 abgepufferte Trägerflüssigkeit und/oder zusätzliche feste Trägermaterialien, dadurch gekennzeichnet, dass man die in Anspruch 1 genannten Massnahmen ausführt.

6. Verfahren zur Herstellung von pharmazeutischen Zusammensetzungen gemäss Anspruch 1 oder 2 enthaltend

a) eine synthetisches, zu mehr als 90 Gew.-% reines Phospholipid der Formel I, worin $R_1$ und $R_2$ gleich sind und 9-cis-Dodecenoyl, 9-cis-Tetradecenoyl, 9-cis-Hexadecenoyl, 9-cis-Octadecenoyl oder 9-cis-Icosenoyl bedeuten, n eins und $Y^{\oplus}$ das Natriumion ist,

b) ein synthetisches, zu mehr als 90 Gew.-% reines Phospholipid der Formel II, worin $R_1$ n-Dodecanoyl, n-Tetradecanoyl, n-Hexadecanoyl oder n-Octadecanoyl, $R_2$ 9-cis-Dodecenoyl, 9-cis-Tetradecenoyl, 9-cis-Hexadecenoyl, 9-cis-Octadecenoyl oder 9-cis-Icosenoyl und $R_a$, $R_b$ und $R_c$ Methyl bedeuten,

c) einen pharmazeutischen Wirkstoff oder ein Stoffgemisch aus der Gruppe der Antiphlogistika, Antibiotika, Antileishmanien, Antimykotika, Antineoplastika and Immunmodulatoren und gegebenenfalls eine auf pH 7,2—7,4 abgepufferte Trägerflüssigkeit, dadurch gekennezeichnet, dass man die in Anspruch 1 genannten Massnahmen ausführt.

7. Verfahren zur Herstellung von pharmazeutischen Zusammensetzungen gemäss Anspruch 3 enthaltend

a) synthetisches, zu mehr als 90 Gew.-% reines Natrium-1,2-di-(9-cis-octadecenoyl)-3-sn-phosphatidyl-(S)-serin,

b) synthetisches, zu mehr als 90 Gew.-% reines 1-n-Hexadecanoyl-2-(9-cis-octadecenoyl)-3-sn-phosphatidylcholin,

c) einen pharmazeutischen Wirkstoff oder ein Stoffgemisch aus der Gruppe der Antiphlogistika, Antibiotika, Antileishmanien, Antimykotika, Antineoplastika and Immunmodulatoren und gegebenenfalls eine auf pH 7,2—7,4 abgepufferte Trägerflüssigkeit, dadurch gekennzeichnet, dass man die in Anspruch 1 genannten Massnahmen ausführt.

8. Verfahren zur Herstellung von pharmazeutischen Zusammensetzungen gemäss Anspruch 4 enthaltend

a) synthetisches, zu mehr als 90 Gew.-% reines Natrium-1,2-di-(9-cis-octadecenoyl)-3-sn-phosphatidyl-(S)-serin,

b) synthetisches, zu mehr als 90 Gew.-% reines 1-n-Hexadecanoyl-2-(9-cis-octadecenoyl)-3-sn-phosphatidylcholin,

c) Diclofenac, Pirprofen, Mitomycin, Cytarabin, Dactinomycin, Daunorubicin, Doxorubicin, Etoposid, N - Acetyl - D - muramyl - L - alanyl - D - isoglutaminyl - L - alanin - 2 - (1,2 - dipalmitoyl - sn - glycero - 3 - hydroxyphosphoryloxy) - äthylamid, N - Acetyl - D - muramyl - L - alanyl - D - glutaminsäure - ($C_{\gamma}$) - L - alanin - 2 - (1,2 - dipalmitoyl - sn - glycero - 3 - hydroxyphosphoryloxy) - äthylamid - dinatriumsalz, N - Acetyl - desmethylmuramyl - L - alanyl - D - isoglutaminnatriumsalz, N - Acetyl - D - muramyl - L - alanyl - D - isoglutamin - natriumsalz, N - Acetyl - D - muramyl - L - alanyl - D - glutamin - α - n - butylester, $N^{\alpha}$ - (N - Acetyl - D - muramyl - L - alanyl - D - isoglutaminyl) - $N^{\varepsilon}$stearoyl - L - lysin oder 6 - O - Stearoyl - N - acetyl - D - muramyl - L - alanin - D - isoglutamin, Lymphokine oder Kombinationen davon, und gegebenenfalls eine auf pH 7,2—7,4 abgepufferte Trägerflüssigkeit, dadurch gekennzeichnet, dass man die in Anspruch 1 genannten Massnahmen ausführt.

9. Verfahren zur Herstellung von pharmazeutischen Zusammensetzungen gemäss Anspruch 5 enthaltend

a) synthetisches, zu mehr als 90 Gew.-% reines Natrium-1,2-di-(9-cis-octadecenoyl)-3-sn-phosphatidyl-(S)-serin,

b) synthetisches, zu mehr als 90 Gew.-% reines 1-n-Hexadecanoyl-2-(9-cis-octadecenoyl)-3-sn-phosphatidylcholin,

c) Mitomycin, Cytarabin, Dactinomycin, Daunorubicin, Doxorubicin, Etoposid, N - Acetyl - D - muramyl - L - alanyl - D - isoglutaminyl - L - alanin - 2 - (1,2 - dipalmitoyl - sn - glycero - 3 - hydroxyphosphoryloxy) - äthylamid, N - Acetyl - desmethylmuramyl - L - alanyl - D - isoglutamin -

natriumsalz oder N - Acetyl - D - muramyl - L - alanyl - D - isoglutamin - natriumsalz, gegebenenfalls in Kombination mit humanem Gamma-Interferon oder Interleukin 2 oder Stoffgemischen, welche in Kulturfiltraten aus Kulturen mit menschlichen T-Lymphozyten aus Milz oder peripherem Blut nach Stimulierung durch Antigene oder Mitogene enthalten und durch hohen Anteil an Makrophagen-aktivierendem Faktor (MAF) gekennzeichnet sind, und gegebenenfalls eine auf pH 7,2—7,4 abgepufferte Trägerflüssigkeit, dadurch gekennzeichnet, dass man die in Anspruch 1 genannten Massnahmen ausführt.

10. Pharmazeutische Zusammensetzungen gemäss Anspruch 6 enthaltend

a) synthetisches, zu mehr als 90 Gew.-% reines Natrium-1,2-di-(9-cis-octadecenoyl)-3-sn-phosphatidyl-(S)-serin,

b) synthetisches, zu mehr als 90 Gew.-% reines 1-n-Hexadecanoyl-2-(9-cis-octadecenoyl)-3-sn-phosphatidylcholin,

c) N - Acetyl - D - muramyl - L - alanyl - D - isoglutaminyl - L - alanin - 2 - (1,2 - dipalmitoyl - sn - glycero - 3 - hydroxyphosphoryloxy) - äthylamid, N - Acetyl - desmethylmuramyl - L - alanyl - D - isoglutamin - natriumsalz oder N - Acetyl - D - muramyl - L - alanyl - D - isoglutamin - natriumsalz, gegebenenfalls in Kombination mit Stoffgemischen, welche in Kulturfiltraten aus Kulturen mit menschlichen T-Lymphozyten aus Milz oder peripherem Blut nach Stimulierung durch Antigene oder Mitogene enthalten und durch mindestens 70%-igen Anteil an MAF gekennzeichnet sind, und gegebenenfalls eine auf pH 7,2—7,4 abgepufferte Trägerflüssigkeit, dadurch gekennzeichnet, dass man die in Anspruch 1 genannten Massnahmen ausführt.

11. Verfahren zur Herstellung von pharmazeutischen Zusammensetzungen gemäss Anspruch 4 enthaltend

a) synthetisches, zu mehr als 90 Gew.-% reines Natrium-1,2-di-(9-cis-octadecenoyl)-3-sn-phosphatidyl-(S)-serin,

b) synthetisches, zu mehr als 90 Gew.-% reines 1-n-Hexadecanoyl-2-(9-cis-octadecenoyl)-3-sn-phosphatidylcholin,

c) N - Acetyl - D - muramyl - L - alanyl - D - isoglutaminyl - L - alanin - 2 - (1,2 - dipalmitoyl - sn - glycero - 3 - hydroxyphosphoryloxy) - äthylamid, und gegebenenfalls eine auf pH 7,2—7,4 abgepufferte Trägerflüssigkeit, dadurch gekennzeichnet, dass man die in Anspruch 1 genannten Verfahrensmassnahmen ausführt.

12. Verfahren zur Herstellung von pharmazeutischen Zusammensetzungen gemäss Anspruch 4 enthaltend

a) synthetisches, zu mehr als 90 Gew.-% reines Natrium-1,2-di-(9-cis-octadecenoyl)-3-sn-phosphatidyl-(S)-serin,

b) synthetisches, zu mehr als 90 Gew.-% reines 1-n-Hexadecanoyl-2-(9-cis-octadecenoyl)-3-sn-phosphatidylcholin,

c) natürliches oder rekombiniertes, humanes Gamma-Interferon und gegebenenfalls eine auf pH 7,2—7,4 abgepufferte Trägerflüssigkeit, dadurch gekennzeichnet, dass man die in Anspruch 1 genannten Massnahmen durchführt.

13. Verfahren zur Herstellung von pharmazeutischen Zusammensetzungen gemäss Anspruch 9 enthaltend

a) synthetisches, zu mehr als 90 Gew.-% reines Natrium-1,2-di-(9-cis-octadecenoyl)-3-sn-phosphatidyl-(S)-serin,

b) synthetisches, zu mehr als 90 Gew.-% reines 1-n-Hexadecanoyl-2-(9-cis-octadecenoyl)-3-sn-phosphatidylcholin,

c) rekombiniertes, humanes Gamma-Interferon mit folgenden Aminosäure-sequenz:

$H_2N$-Cys-Tyr-Cys-Gln-Asp-Pro-Tyr-Val-Gln-Glu-Ala-Glu-Asn-Leu-Lys-Lys-
Tyr-Phe-Asn-Ala-Gly-His-Ser-Asp-Val-Ala-Asp-Asn-Gly-Thr-Leu-Phe-Leu-
Gly-Ile-Leu-Lys-Asn-Trp-Lys-Glu-Glu-Ser-Asp-Arg-Lys-Ile-Met-Gln-Ser-
Gln-Ile-Val-Ser-Phe-Tyr-Phe-Lys-Leu-Phe-Lys-Asn-Phe-Lys-Asp-Asp-Gln-
Ser-Ile-Gln-Lys-Ser-Val-Glu-Thr-Ile-Lys-Glu-Asp-Met-Asn-Val-Lys-Phe-
Phe-Asn-Ser-Asn-Lys-Lys-Lys-Arg-Asp-Asp-Phe-Glu-Lys-Leu-Thr-Asn-Tyr-
Ser-Val-Thr-Asp-Leu-Asn-Val-Gln-Arg-Lys-Ala-Ile-His-Glu-Leu-Ile-Gln-
Val-Met-Ala-Glu-Leu-Ser-Pro-Ala-Ala-Lys-Thr-Glu-Lys-Arg-Lys-Arg-Ser-
Gln-Met-Leu-Phe-Gln-Gly-Arg-Arg-Ala-Ser-Gln-OH

und gegebenenfalls eine auf pH 7,2—7,4 abgepufferte Trägerflüssigkeit, dadurch gekennzeichnet, dass man die in Anspruch 1 genannten Massnahmen durchführt.

14. Verfahren zur Herstellung von pharmazeutischen Zusammensetzungen gemäss Anspruch 9 enthaltend

a) synthetisches, zu mehr als 90 Gew.-% reines Natrium-1,2-di-(9-cis-octadecenoyl)-3-sn-phosphatidyl-(S)-serin,

b) synthetisches, zu mehr als 90 Gew.-% reines 1-n-Hexadecanoyl-2-(9-cis-octadecenoyl)-3-sn-phosphatidylcholin,

c) rekombiniertes, humanes Gamma-Interferon mit folgenden Aminosäure-sequenz:

$H_2N$-Cys-Tyr-Cys-Gln-Asp-Pro-Tyr-Val-Lys-Glu-Ala-
Glu-Asn-Leu-Lys-Lys-Tyr-Phe-Asn-Ala-Gly-His-Ser-
Asp-Val-Ala-Asp-Asn-Gly-Thr-Leu-Phe-Leu-Gly-Ile-
Leu-Lys-Asn-Trp-Lys-Glu-Glu-Ser-Asp-Arg-Lys-Ile-
Met-Gln-Ser-Gln-Ile-Val-Ser-Phe-Tyr-Phe-Lys-Leu-
Phe-Lys-Asn-Phe-Lys-Asp-Asp-Gln-Ser-Ile-Gln-Lys-
Ser-Val-Glu-Thr-Ile-Lys-Glu-Asp-Met-Asn-Val-Lys-
Phe-Phe-Asn-Ser-Asn-Lys-Lys-Lys-Arg-Asp-Asp-Phe-
Glu-Lys-Leu-Thr-Asn-Tyr-Ser-Val-Thr-Asp-Leu-Asn-
Val-Gln-Arg-Lys-Ala-Ile-His-Glu-Leu-Ile-Gln-Val-
Met-Ala-Glu-Leu-Ser-Pro-Ala-Ala-Lys-Thr-Gly-Lys-
Arg-Lys-Arg-Ser-Gln-Met-Leu-Phe-Arg-Gly-Arg-Arg-
Ala-Ser-Gln-OH

und gegebenenfalls eine auf pH 7,2—7,4 abgepufferte Trägerflüssigkeit, dadurch gekennzeichnet, dass man die in Anspruch 1 genannten Massnahmen durchführt.

15. Verfahren gemäss Anspruch 1, dadurch gekennzeichnet, dass man die homogene Mischung durch Lyophilisatbildung herstellt.

16. Verfahren gemäss Ansprüche 1, dadurch gekennzeichnet, dass man die Dispersion bei Temperaturen unterhalb 30° durchführt.

17. Verfahren gemäss Ansprüche 12, dadurch gekennzeichnet, dass man für die Herstellung des Lyophilisats tert-Butanol als Lösungsmittel verwendet.

18. Mischungen bestehend aus synthetischen, zu mehr als 90 Gew.-% reinen Phospholipiden der Formeln I und II gemäss Anspruch 1.

**Revendications pour les États contractants: BE CH DE FR GB IT LI LU NL SE**

1. Compositions pharmaceutiques contenant

a) un phospholipide synthétique essentiellement pur de formule

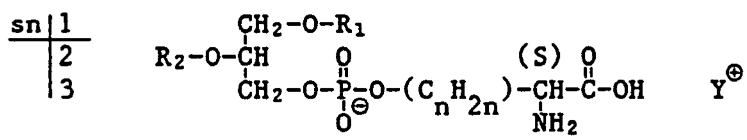

(I),

où $R_1$ et $R_2$ représentent indépendamment l'un de l'autre un alcénoyle en $C_{10-20}$ avec un nombre pair d'atomes de carbone, n un nombre entier allant de 1 à 3 et $Y^{\oplus}$ le cation d'une base pharmaceutiquement acceptable,

b) un essentiellement pur de formule

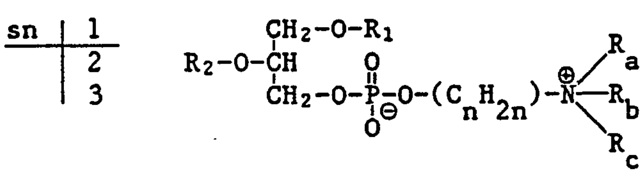

(II),

où $R_1$ représente un alcanoyle en $C_{10-20}$ avec un nombre pair d'atomes de carbone, $R_2$ un alcénoyle en

$C_{10-20}$ ayant un nombre pair d'atomes de carbone, $R_a$, $R_b$ et $R_c$ représentent un hydrogène ou un alcoyle en $C_{1-4}$ et n un nombre entier allant de 2 à 4,

c) un corps ou un mélange de corps ayant une action biologique et éventuellement un liquide support et/ou des supports solides additionnels.

2. Compositions pharmaceutiques contenant des phospholipides synthétiques purs à plus de 90% en poids dans un rapport de mélange des phospholipides de formule I aux phospholipides de formule II d'environ 10 à 90 à environ 50 pour 50% molaire et une substance pharmaceutique ou un mélange de substances pharmaceutiques actives et éventuellement un liquide support tamponné à pH 7,0—7,8 et/ou des supports solides supplémentaires.

3. Compositions pharmaceutiques selon la revendication 1 ou 2, contenant

a) un phospholipide de formule I de synthèse pur à plus de 90%, où $R_1$ et $R_2$ sont identiques et représentent un 9-cis-dodécénoyle, 9-cis-tétradécénoyle, 9-cis-hexadécénoyle, 9-cis-octadécénoyle ou 9-cis-icosénoyle, n vaut 1 et $Y^{\oplus}$ est l'ion sodium,

b) un phospholipide de formule II synthétique, pur à plus de 90%, où $R_1$ représente un n-dodécanoyle, n-tétradécanoyle, n-hexadécanoyle, ou n-octadécanoyl, $R_2$ un 9-cis-dodécénoyle, 9-cis-tétradécénoyle, 9-cis-hexadécénoyle, 9-cis-octadécénoyle ou 9-cis-icosénoyle, et $R_a$, $R_b$ et $R_c$ un méthyle,

c) une substance active pharmaceutique ou un mélange de substance actives pharmaceutiques et éventuellement un support liquide tamponné à pH 7,2—7,4.

4. Compositions selon la revendication 3, contenant

a) la sodium - 1,2 - di - (9 - cis - octadécénoyl) - 3 - sn - phosphatidyl - (S) - sérine synthétique, pure à plus de 90% en poids,

b) la 1 - n - hexadécanoyl - 2 - (9 - cis - octadécénoyl) - 3 - sn - phosphatidylcholine synthétique, pure à plus de 90% en poids,

c) un substance active pharmaceutique ou un mélange de substances actives pharmaceutiques et éventuellement un liquide-support tamponné à pH 7,2—7,4.

5. Compositions pharmaceutiques selon la revendication 1, contenant des phospholipides synthétiques purs à plus de 90% en poids dans un rapport de mélange des phospholipides de formule I aux phospholipides de formule II d'environ 10 pour 90 à environ 50 pour 50% molaire et une substance active pharmaceutique ou un mélange de corps du groupe des antiinflammatoires, antibiotiques, antileishmaniens, antimycotiques, antinéoplasiques, et immunomodulateurs et éventuellement un liquide-support tamponné à pH 7,0—7,8 et/ou des supports solides additionnels.

6. Compositions pharmaceutiques selon la revendication 1 ou 2, contenant

a) un phospholipide de formule I pur à plus de 90% en poids synthétique, où $R_1$ et $R_2$ sont identiques et représentent le 9-cis-dodécénoyle, le 9-cis-tétradécénoyle, le 9-cis-hexadécénoyle, le 9-cis-octadécénoyle ou le 9-cis-icosénoyle, n vaut 1 et $Y^{\oplus}$ est l'ion sodium,

b) un phospholipide de formule II synthétique, pur à plus de 90% en poids, où $R_1$ représente un n-dodécanoyle, n-tétradécanoyle, n-hexadécanoyle, ou un n-octadécanoyl, $R_2$ représente un 9-cis-dodécénoyle, 9-cis-tétradécénoyle, 9-cis-hexadécénoyle, 9-cis-octadécénoyle, ou 9-cis-icosénoyle et $R_a$, $R_b$ et $R_c$ un méthyle,

c) une substance active pharmaceutiquement ou un mélange de corps du groupe des antiinflammatoires, antibiotiques, antileishmaniens, antimycotiques, antinéoplasiques et immunomodulateurs et éventuellement un liquide-support tamponné à pH 7,2—7,4.

7. Compositions pharmaceutiques selon la revendication 3 contenant

a) la sodium - 1,2 - di - (9 - cis - octadécénoyl) - 3 - sn - phosphatidyl - (S) - sérine synthétique, pure à plus de 90% en poids,

b) la 1 - n - hexadécanoyl - 2 - (9 - cis - octadécénoyl) - 3 - sn - phosphatidylcholine synthétique, pure à plus de 90% en poids,

c) un substance active pharmaceutique ou un mélange de corps du groupe des antiinflammatoires, antibiotiques, antileishmaniens, antimycotiques, antinéoplasiques et immunomodulateurs et éventuellement un liquide-support tamponné à pH 7,2—7,4.

8. Compositions pharmaceutiques selon la revendication 4 contenant

a) la sodium - 1,2 - di - (9 - cis - octadécénoyl) - 3 - sn - phosphatidyl - (S) - sérine synthétique, pure à plus de 90% en poids,

b) la 1 - n - hexadécanoyl - 2 - (9 - cis - octadécénoyl) - 3 - sn - phosphatidylcholine synthétique, pure à plus de 90% en poids,

c) un substance active pharmaceutique ou un mélange de corps du groupe diclofénac, pirprofen, mitomycine, cytarabine, dactinomycine, daunorubicine, doxorubicine, étoposide, N - acétyl - D - muramyl - L - alanyl - D - isoglutaminyl - L - alanin - 2 - (1,2 - dipalmitoyl - sn - glycéro - 3 - hydroxyphosphoryloxy) - éthylamide, sel de sodium de $(C_{\gamma})$ - L - alanin - 2 - (1,2 - dipalmitoyl - sn - glycéro - 3 - hydroxyphosphoryloxy) - éthylamide de l'acide N - acétyl - D - muramyl - L - alanyl - D - glutamique, sel de sodium de N - acétyl - D - muramyl - L - alanyl - D - isoglutamine, sel de sodium de N - acétyl - D - muramyl - L - alanyl - D - isoglutamine, α - n - butylester de N - acétyl - D - muramyl - L - alanyl - D - glutamine, $N^{\alpha}$ - (N - acétyl - D - muramyl - L - alanyl - D - isoglutaminyl) - $N^{\varepsilon}$-stéaroyl - L - lysine ou 6 - O - stéaroyl - N - acétyl - D - muramyl - L - alanyl - D - isoglutamine, des lymphokines ou leurs combinaisons et éventuellement un liquide-support tamponné à pH 7,2—7,4.

9. Compositions pharmaceutiques selon la revendication 4 contenant

a) la sodium - 1,2 - di - (9 - cis - octadécénoyl) - 3 - sn - phosphatidyl - (S) - sérine synthétique, pure à plus de 90% en poids,

b) la 1 - n - hexadécanoyl - 2 - (9 - cis - octadécénoyl) - 3 - sn - phosphatidylcholine synthétique, pure à plus de 90% en poids,

c) un substance active pharmaceutique du groupe mitomycine, cytarabine, dactinomycine, daunorubicin, doxorubicine, etoposide, N - acétyl - D - muramyl - L - alanyl - D - isoglutaminyl - L - alanin - 2 - (1,2 - dipalmitoyl - sn - glycéro - 3 - hydroxyphosphoryloxy) - éthylamide, sel de sodium de N - acétyl - desméthylmuramyl - L - alanyl - D - isoglutamine ou sel de sodium de N - acétyl - D - muramyl - L - alanyl - D - isoglutamine, éventuellement en combinaison avec de l'Interféron-Gamma humain ou de l'Interleukine 2 ou des mélanges de corps que l'on obtient dans des filtrats de culture à partir de cultures avec les lymphocytes T humains de rate ou de sang périphérique après stimulation avec des antigènes ou des mitogènes et qui se caractérisent par une proportion élevée de facteur activant les macrophages (MAF) et éventuellement un liquide-support tamponné à pH 7,2—7,4.

10. Compositions pharmaceutiques selon la revendication 4, contenant

a) la sodium - 1,2 - di - (9 - cis - octadécénoyl) - 3 - sn - phosphatidyl - (S) - sérine synthétique, pure à plus de 90% en poids,

b) la 1 - n - hexadécanoyl - 2 - (9 - cis - octadécénoyl) - 3 - sn - phosphatidylcholine, synthétique, pure à plus de 90% en poids,

c) le N - acétyl - D - muramyl - L - alanyl - D - isoglutaminyl - L - alanin - 2 - (1,2 - dipalmitoyl - sn - glycéro - 3 - hydroxyphosphoryloxy) - éthylamide, le sel de sodium de N - acétyl - desméthylmuramyl - L - alanyl - D - isoglutamine ou le sel de sodium de N - acétyl - D - muramyl - L - alanyl - D - isoglutamine, éventuellement en combinaison avec des mélanges de corps que l'on obtient dans des filtrats de culture provenant de cultures avec des lymphocytes T humains de rate ou de sang périphérique après stimulation par des antigènes ou des mitogènes et qui se caractérisent par une proportion d'au moins 70% de MAF, et éventuellement un liquide-support tamponné à pH 7,2—7,4.

11. Compositions pharmaceutiques selon la revendication 4 contenant

a) la sodium - 1,2 - di - (9 - cis - octadécénoyl) - 3 - sn - phosphatidyl - (S) - sérine synthétique, pure à plus de 90% en poids,

b) la 1 - n - hexadécanoyl - 2 - (9 - cis - octadécénoyl) - 3 - sn - phosphatidylcholine, synthétique, pure à plus de 90% en poids,

c) le N - acétyl - D - muramyl - L - alanyl - D - isoglutaminyl - L - alanin - 2 - (1,2 - dipalmitoyl - sn - glycéro - 3 - hydroxyphosphoryloxy) - éthylamide, et éventuellement un liquide-support tamponné à pH 7,2—7,4.

12. Compositions pharmaceutiques selon la revendication 4, contenant

a) la sodium - 1,2 - di - (9 - cis - octadécénoyl) - 3 - sn - phosphatidyl - (S) - sérine synthétique, à plus de 90% en poids,

b) la 1 - n - hexadécanoyl - 2 - (9 - cis - octadécénoyl) - 3 - sn - phosphatidylcholine, synthétique, à plus de 90% en poids,

c) l'Interféron-Gamma humain naturel ou recombinant et éventuellement un liquide-support tamponné à pH 7,2—7,4.

13. Compositions pharmaceutiques selon la revendication 9 contenant

a) la sodium - 1,2 - di - (9 - cis - octadécénoyl) - 3 - sn - phosphatidyl - (S) - sérine synthétique, à plus de 90% en poids,

b) la 1 - n - hexadécanoyl - 2 - (9 - cis - octadécénoyl) - 3 - sn - phosphatidylcholine, synthétique, pure à plus de 90% en poids,

c) l'Interferon-Gamma humain recombiné ayant la séquence d'acides aminés suivante:

$H_2N$-Cys-Tyr-Cys-Gln-Asp-Pro-Tyr-Val-Gln-Glu-Ala-Glu-Asn-Leu-Lys-Lys-
Tyr-Phe-Asn-Ala-Gly-His-Ser-Asp-Val-Ala-Asp-Asn-Gly-Thr-Leu-Phe-Leu-
Gly-Ile-Leu-Lys-Asn-Trp-Lys-Glu-Glu-Ser-Asp-Arg-Lys-Ile-Met-Gln-Ser-
Gln-Ile-Val-Ser-Phe-Tyr-Phe-Lys-Leu-Phe-Lys-Asn-Phe-Lys-Asp-Asp-Gln-
Ser-Ile-Gln-Lys-Ser-Val-Glu-Thr-Ile-Lys-Glu-Asp-Met-Asn-Val-Lys-Phe-
Phe-Asn-Ser-Asn-Lys-Lys-Lys-Arg-Asp-Asp-Phe-Glu-Lys-Leu-Thr-Asn-Tyr-
Ser-Val-Thr-Asp-Leu-Asn-Val-Gln-Arg-Lys-Ala-Ile-His-Glu-Leu-Ile-Gln-
Val-Met-Ala-Glu-Leu-Ser-Pro-Ala-Ala-Lys-Thr-Glu-Lys-Arg-Lys-Arg-Ser-
Gln-Met-Leu-Phe-Gln-Gly-Arg-Arg-Ala-Ser-Gln-OH

et éventuellement un liquide-support tamponné à pH 7,2—7,4.

14. Compositions pharmaceutiques selon la revendication 9, contenant

a) la sodium - 1,2 - di - (9 - cis - octadécénoyl) - 3 - sn - phosphatidyl - (S) - sérine synthétique, pure à plus de 90% en poids,

b) la 1 - n - hexadécanoyl - 2 - (9 - cis - octadécénoyl) - 3 - sn - phosphatidylcholine, synthétique, pure à plus de 90% en poids,

c) l'Interferon-Gamma humain recombinant, ayant la séquence d'acides aminés suivante:

$H_2N$-Cys-Tyr-Cys-Gln-Asp-Pro-Tyr-Val-Lys-Glu-Ala-
Glu-Asn-Leu-Lys-Lys-Tyr-Phe-Asn-Ala-Gly-His-Ser-
Asp-Val-Ala-Asp-Asn-Gly-Thr-Leu-Phe-Leu-Gly-Ile-
Leu-Lys-Asn-Trp-Lys-Glu-Glu-Ser-Asp-Arg-Lys-Ile-
Met-Gln-Ser-Gln-Ile-Val-Ser-Phe-Tyr-Phe-Lys-Leu-
Phe-Lys-Asn-Phe-Lys-Asp-Asp-Gln-Ser-Ile-Gln-Lys-
Ser-Val-Glu-Thr-Ile-Lys-Glu-Asp-Met-Asn-Val-Lys-
Phe-Phe-Asn-Ser-Asn-Lys-Lys-Lys-Arg-Asp-Asp-Phe-
Glu-Lys-Leu-Thr-Asn-Tyr-Ser-Val-Thr-Asp-Leu-Asn-
Val-Gln-Arg-Lys-Ala-Ile-His-Glu-Leu-Ile-Gln-Val-
Met-Ala-Glu-Leu-Ser-Pro-Ala-Ala-Lys-Thr-Gly-Lys-
Arg-Lys-Arg-Ser-Gln-Met-Leu-Phe-Arg-Gly-Arg-Arg-
Ala-Ser-Gln-OH

et éventuellement un liquide-support tamponné à pH 7,2—7,4.

15. Mélanges convenant pour la préparation de liposomes constitués de phospholipides, synthétiques, purs à plus de 90% en poids de formules I et II selon la revendication I.

16. Procédé de préparation de compositions pharmaceutiques selon l'une des revendications 1—11 caractérisé en ce que

a) on prépare un mélange homogène constitué de phospholipides de formules I et II synthétiques, purs à plus de 90% en poids, et d'un corps lipophile ou d'un mélange de corps, ayant une action biologique et en ce que pour la préparation des liposomes on disperse le mélange homogène obtenu en phase aqueuse ou

b) on prépare un mélange homogène constitué de phospholipides de formules I et II synthétiques purs à plus de 90% en poids, et pour préparer des liposomes en ce qu'on disperse le mélange homogène apparu en phase aqueuse contenant un corps soluble dans l'eau ou un mélange de corps ayant une action biologique, et, si nécessaire, en ce qu'on tamponne la dispersion aqueuse obtenue à pH 7,0—7,8 et, si on le désire, on concentre et/ou sépare les liposomes obtenus.

17. Composition pharmaceutique selon la revendication 1 aux fins d'application dans le traitement du corps humain on animal.

**Revendications pour l'Etats contractant: AT**

1. Procédé de préparation de compositions pharmaceutiques contenant

a) un phospholipide synthétique essentiellement pur de formule

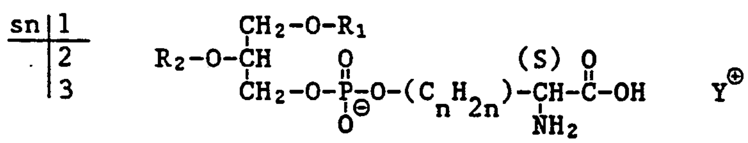

(I),

où $R_1$ et $R_2$ représentent indépendamment l'un de l'autre un alcénoyle en $C_{10-20}$ avec un nombre pair d'atomes de carbone, n un nombre entier allant de 1 à 3 et $Y^{\oplus}$ le cation d'une base pharmaceutiquement acceptable,

b) un phospholipide essentiellement pur de formule

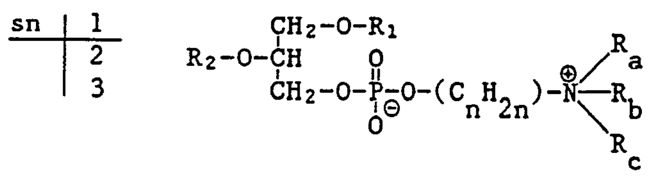

(II),

où $R_1$ représente un alcanoyle en $C_{10-20}$ avec un nombre pair d'atomes de carbone, $R_2$ un alcénoyle en $C_{10-20}$ ayant un nombre pair d'atomes de carbone, $R_a$, $R_b$ et $R_c$ représentent un hydrogène ou un alcoyle en $C_{1-4}$ et n un nombre entier allant de 2 à 4,

c) un corps ou un mélange de corps ayant une action biologique et éventuellement un liquide support et/ou des supports solides additionnels, caractérisé en ce que

a) on prépare un mélange homogène constitué de phospholipides de formules I et II synthétiques, pors à plus de 90% en poids, et d'un corps lipophile ou d'un mélange de corps, ayant une action biologique et en ce que pour la préparation des liposomes on disperse le mélange homogène obtenu en phase aqueuse ou

b) on prépare un mélange homogène constitué de phospholipides de formules I et II synthétiques purs à plus de 90% en poids, et pour préparer des liposomes en ce qu'on disperse le mélange homogène apparu en phase aqueuse contenant un corps soluble dans l'eau ou un mélange de corps ayant une action biologique, et, si nécessaire, en ce qu'on tamponne la dispersion aqueuse obtenue à pH 7,0—7,8 et, si on le désire, on concentre et/ou sépare les liposomes obtenus.

2. Procédé de préparation de compositions pharmaceutiques selon la revendication 1, contenant des phospholipides synthétiques purs à plus de 90% en poids dans un rapport de mélange des phospholipides de formule I aux phospholipides de formule II d'environ 10 à 90 à environ 50 pour 50% molaire et une substance pharmaceutique ou un mélange de substances pharmaceutiques actives et éventuellement un liquide support tamponné à pH 7,0—7,8 et/ou des supports solides supplémentaires, caractérisé en ce qu'on prend les mesures mentionnées dans la revendication 1.

3. Procédé de préparation de compositions pharmaceutiques selon la revendication 1 ou la revendication 2, contenant

a) un phospholipide de formule I pur à plus de 90% en poids, synthetique, où $R_1$ et $R_2$ sont identiques et représentent le 9-cis-dodécénoyle, le 9-cis-tétradécénoyle, le 9-cis-hexadécénoyle, le 9-cis-octadécénoyle ou le 9-cis-icosénoyle, n vaut 1 et $Y^{\oplus}$ est l'ion sodium,

b) un phospholipide de formule II synthétique, pur à plus de 90% en poids, où $R_1$ représente un n-dodécanoyle, n-tétradécanoyle, n-hexadécanoyle, ou un n-octadécanoyl, $R_2$ représente un 9-cis-dodécénoyle, 9-cis-tétradécénoyle, 9-cis-hexadécénoyle, 9-cis-octadécénoyle ou un 9-cis-icosénoyle et $R_a$, $R_b$ et $R_c$ un méthyle,

c) une substance active pharmaceutique ou un mélange de substances actives pharmaceutiques et éventuellement un liquide-support tamponné à pH 7,2—7,4, caractérisé en ce qu'on prend les mesures mentionnées dans la revendication 1.

4. Procédé de préparation de compositions pharmaceutiques selon la revendication 3 contenant

a) la sodium - 1,2 - di - (9 - cis - octadécénoyl) - 3 - sn - phosphatidyl - (S) - sérine synthétique, pure à plus de 90% en poids,

b) la 1 - n - hexadécanoyl - 2 - (9 - cis - octadécénoyl) - 3 - sn - phosphatidylcholine synthétique, pure à plus de 90% en poids,

c) une substance active pharmaceutique ou éventuellement un liquide-support tamponné à pH 7,2—7,4, caractérisé en ce qu'on prend les mesures mentionnées dans la revendication 1.

5. Procédé de préparation de compositions pharmaceutiques selon la revendication 1 contenant des phospholipides synthétiques, purs à plus de 90% en poids, dans un rapport de mélange des phospholipides de formule I aux phospholipides de formule II d'environ 10 pour 90 à environ 50 pour 50% molaire et une substance active pharmaceutique ou un mélange substances actives pharmaceutiques du groupe des antiinflammatoires, antibiotiques, antileishmaniens, antimycotiques, antinéoplasiques, et immunomodulateurs et éventuellement un liquide-support tamponné à pH 7,0—7,8 et/ou des supports solides additionnels, caractérisé en ce qu'on prend les mesures mentionnées dans la revendication 1.

6. Procédé de préparation de compositions pharmaceutiques selon la revendication 1 ou 2, contenant

a) un phospholipide de formule I synthétique pur à plus de 90% en poids, où $R_1$ et $R_2$ sont identiques et représentent un 9-cis-dodécénoyle, 9-cis-tétradécénoyle, 9-cis-hexadécénoyle, 9-cis-octadécénoyle ou 9-cis-icosénoyle, n vaut 1 et $Y^{\oplus}$ est l'ion sodium,

b) un phospholipide de formule II synthétique, pur à plus de 90% en poids, où $R_1$ représente un n-dodécanoyle, n-tétradécanoyle, n-hexadécanoyle ou n-octadécanoyl, $R_2$ un 9-cis-dodécénoyle, 9-cis-tétradécénoyle, 9-cis-hexadécénoyle, 9-cis-octadécénoyle, ou 9-cis-icosénoyle et $R_a$, $R_b$ et $R_c$ represéntent un méthyle,

c) une substance active pharmaceutique ou un mélange de substances actives pharmaceutiques du groupe des antiinflammatoires, antibiotiques, antileishmaniens, antimycotiques, antinéoplasiques, ou

immunomodulateurs et éventuellement un liquide-support tamponné à pH 7,2—7,4, caractérisé en ce qu'on prend les mesures mentionnées dans la revendication 1.

7. Procédé de préparation de compositions pharmaceutiques selon la revendication 3, contenant

a) la sodium - 1,2 - di - (9 - cis - octadécénoyl) - 3 - sn - phosphatidyl - (S) - sérine, synthétique, pure à plus de 90% en poids,

b) la 1 - n - hexadécanoyl - 2 - (9 - cis - octadécénoyl) - 3 - sn - phosphatidylcholine, synthétique, pure à plus de 90% en poids,

c) une substance active ou mélange de substances actives de groupe des antiinflammatoires, antibiotiques, antileishmaniens, antimycotiques, antinéoplasiques et immunomodulateurs et éventuellement un liquide-support tamponné à pH 7,2—7,4, caractérisé en ce qu'on prend les mesures mentionnées dans la revendication 1.

8. Procédé de préparation de compositions pharmaceutiques selon la revendication 4 contenant

a) la sodium - 1,2 - di - (9 - cis - octadécénoyl) - 3 - sn - phosphatidyl - (S) - sérine synthétique, pure à plus de 90% en poids,

b) la 1 - n - hexadécanoyl - 2 - (9 - cis - octadécénoyl) - 3 - sn - phosphatidylcholine synthétique, pure à plus de 90% en poids,

c) une substance active pharmaceutique ou un mélange de corps du groupe diclofénac, pirprofen, mitomycine, cytarabine, dactinomycine, daunorubicine, doxorubicine, étoposide, N - acétyl - D - muramyl - L - alanyl - D - isoglutaminyl - L - alanin - 2 - (1,2 - dipalmitoyl - sn - glycéro - 3 - hydroxyphosphoryloxy) - éthylamide, sel de sodium de ($C_\gamma$) - L - alanin - 2 - (1,2 - dipalmitoyl - sn - glycéro - 3 - hydroxyphosphoryloxy) - éthylamide de l'acide N - acétyl - D - muramyl - L - alanyl - D - glutamique, sel de sodium de N - acétyl - D - muramyl - L - alanyl - D - isoglutamine, sel de sodium de N - acétyl - D - muramyl - L - alanyl - D - isoglutamine, α - n - butylester de N - acétyl - D - muramyl - L - alanyl - D - glutamine, $N^\alpha$ - (N - acétyl - D - muramyl - L - alanyl - D - isoglutaminyl) - $N^\varepsilon$-stéaroyl - L - lysine ou 6 - O - stéaroyl - N - acétyl - D - muramyl - L - alanyl - D - isoglutamine, des lymphokines ou leurs combinaisons et éventuellement un liquide-support tamponné à pH 7,2—7,4, caractérisé en ce qu'on prend les mesures mentionnées dans la revendication 1.

9. Procédé de préparation de compositions pharmaceutiques selon la revendication 5, contenant

a) la sodium - 1,2 - di - (9 - cis - octadécénoyl) - 3 - sn - phosphatidyl - (S) - sérine synthétique, pure à plus de 90% en poids,

b) la 1 - n - hexadécanoyl - 2 - (9 - cis - octadécénoyl) - 3 - sn - phosphatidylcholine, synthétique, pure à plus de 90% en poids,

c) la mitomycine, cytarabine, dactinomycine, daunorubicine, doxorubicine, etoposide, N - acétyl - D - muramyl - L - alanyl - D - isoglutaminyl - L - alanin - 2 - (1,2 - dipalmitoyl - sn - glycéro - 3 - hydroxyphosphoryloxy) - éthylamide, sel de sodium de N - acétyl - desméthylmuramyl - L - alanyl - D - isoglutamine ou sel de sodium de N - acétyl - D - muramyl - L - alanyl - D - isoglutamine, éventuellement en combinaison avec de l'Interféron-Gamma humain ou de l'Interleukine 2 ou des mélanges de corps que l'on obtient dans des filtrats de culture à partir de cultures avec les lymphocytes T humains de rate ou de sang périphérique après stimulation avec des antigènes ou des mitogènes et qui se caractérisent par une proportion élevée de facteur activant les macrophages (MAF) et éventuellement un liquide-support tamponné à pH 7,2—7,4, caractérisé en ce qu'on prend les mesures mentionnées dans la revendication 1.

10. Compositions pharmaceutiques selon la revendication 6, contenant

a) la sodium - 1,2 - di - (9 - cis - octadécénoyl) - 3 - sn - phosphatidyl - (S) - sérine synthétique, pure à plus de 90% en poids,

b) la 1 - n - hexadécanoyl - 2 - (9 - cis - octadécénoyl) - 3 - sn - phosphatidylcholine, synthétique, pure à plus de 90% en poids,

c) le N - acétyl - D - muramyl - L - alanyl - D - isoglutaminyl - L - alanin - 2 - (1,2 - dipalmitoyl - sn - glycéro - 3 - hydroxyphosphoryloxy) - éthylamide, le sel de sodium de N - acétyl - desméthylmuramyl - L - alanyl - D - isoglutamine ou le sel de sodium de N - acétyl - D - muramyl - L - alanyl - D - isoglutamine, éventuellement en combinaison avec des mélanges de corps que l'on obtient dans des filtrats de culture provenant de cultures avec des lymphocytes T humains de rate ou de sang périphérique après stimulation par des antigènes ou des mitogènes et qui se caractérisent par une proportion d'au moins 70% de MAF, et éventuellement un liquide-support tamponné à pH 7,2—7,4, caractérisé en ce qu'on prend les mesures mentionnées dans la revendication 1.

11. Procédé de préparation de compositions pharmaceutiques selon la revendication 4 contenant

a) la sodium - 1,2 - di - (9 - cis - octadécénoyl) - 3 - sn - phosphatidyl - (S) - sérine synthétique, pure à plus de 90% en poids,

b) la 1 - n - hexadécanoyl - 2 - (9 - cis - octadécénoyl) - 3 - sn - phosphatidylcholine, synthétique, pure à plus de 90% en poids,

c) le N - acétyl - D - muramyl - L - alanyl - D - isoglutaminyl - L - alanin - 2 - (1,2 - dipalmitoyl - sn - glycéro - 3 - hydroxyphosphoryloxy) - éthylamide, et éventuellement un liquide-support tamponné à pH 7,2—7,4, caractérisé en ce qu'on prend les mesures mentionnées dans la revendication 1.

12. Procédé de préparation de compositions pharmaceutiques selon la revendication 4 contenant

a) la sodium - 1,2 - di - (9 - cis - octadécénoyl) - 3 - sn - phosphatidyl - (S) - sérine, synthétique, pure à plus de 90% en poids,

b) la 1 - n - hexadécanoyl - 2 - (9 - cis - octadécénoyl) - 3 - sn - phosphatidylcholine, synthétique, pure à plus de 90% en poids,

c) l'Interferon-Gamma humain naturel ou recombinant et éventuellement un liquide-support tamponné à pH 7,2—7,4, caractérisé en ce qu'on prend les mesures mentionnées dans la revendication 1.

13. Procédé de préparation de compositions pharmaceutiques selon la revendication 9 contenant

a) la sodium - 1,2 - di - (9 - cis - octadécénoyl) - 3 - sn - phosphatidyl - (S) - sérine synthétique, à plus de 90% en poids,

b) la 1 - n - hexadécanoyl - 2 - (9 - cis - octadécénoyl) - 3 - sn - phosphatidylcholine, synthétique, pure à plus de 90% en poids,

c) l'Interferon-Gamma humain recombiné ayant la séquence d'acides aminés suivante:

$H_2N$-Cys-Tyr-Cys-Gln-Asp-Pro-Tyr-Val-Gln-Glu-Ala-Glu-Asn-Leu-Lys-Lys-
Tyr-Phe-Asn-Ala-Gly-His-Ser-Asp-Val-Ala-Asp-Asn-Gly-Thr-Leu-Phe-Leu-
Gly-Ile-Leu-Lys-Asn-Trp-Lys-Glu-Glu-Ser-Asp-Arg-Lys-Ile-Met-Gln-Ser-
Gln-Ile-Val-Ser-Phe-Tyr-Phe-Lys-Leu-Phe-Lys-Asn-Phe-Lys-Asp-Asp-Gln-
Ser-Ile-Gln-Lys-Ser-Val-Glu-Thr-Ile-Lys-Glu-Asp-Met-Asn-Val-Lys-Phe-
Phe-Asn-Ser-Asn-Lys-Lys-Lys-Arg-Asp-Asp-Phe-Glu-Lys-Leu-Thr-Asn-Tyr-
Ser-Val-Thr-Asp-Leu-Asn-Val-Gln-Arg-Lys-Ala-Ile-His-Glu-Leu-Ile-Gln-
Val-Met-Ala-Glu-Leu-Ser-Pro-Ala-Ala-Lys-Thr-Glu-Lys-Arg-Lys-Arg-Ser-
Gln-Met-Leu-Phe-Gln-Gly-Arg-Arg-Ala-Ser-Gln-OH

et éventuellement un liquide-support tamponné à pH 7,2—7,4, caractérisé en ce qu'on prend les mesures mentionnées dans la revendication 1.

14. Procédé de préparation de compositions pharmaceutiques selon la revendication 9, contenant

a) la sodium - 1,2 - di - (9 - cis - octadécénoyl) - 3 - sn - phosphatidyl - (S) - sérine synthétique, à plus de 90% en poids,

b) la 1 - n - hexadécanoyl - 2 - (9 - cis - octadécénoyl) - 3 - sn - phosphatidylcholine, synthétique, à plus de 90% en poids,

c) l'Interferon-Gamma humain recombinant, ayant la séquence d'acides aminés suivante:

$H_2N$-Cys-Tyr-Cys-Gln-Asp-Pro-Tyr-Val-Lys-Glu-Ala-
Glu-Asn-Leu-Lys-Lys-Tyr-Phe-Asn-Ala-Gly-His-Ser-
Asp-Val-Ala-Asp-Asn-Gly-Thr-Leu-Phe-Leu-Gly-Ile-
Leu-Lys-Asn-Trp-Lys-Glu-Glu-Ser-Asp-Arg-Lys-Ile-
Met-Gln-Ser-Gln-Ile-Val-Ser-Phe-Tyr-Phe-Lys-Leu-
Phe-Lys-Asn-Phe-Lys-Asp-Asp-Gln-Ser-Ile-Gln-Lys-
Ser-Val-Glu-Thr-Ile-Lys-Glu-Asp-Met-Asn-Val-Lys-
Phe-Phe-Asn-Ser-Asn-Lys-Lys-Lys-Arg-Asp-Asp-Phe-
Glu-Lys-Leu-Thr-Asn-Tyr-Ser-Val-Thr-Asp-Leu-Asn-
Val-Gln-Arg-Lys-Ala-Ile-His-Glu-Leu-Ile-Gln-Val-
Met-Ala-Glu-Leu-Ser-Pro-Ala-Ala-Lys-Thr-Gly-Lys-
Arg-Lys-Arg-Ser-Gln-Met-Leu-Phe-Arg-Gly-Arg-Arg-
Ala-Ser-Gln-OH

et éventuellement un liquide-support tamponné à pH 7,2—7,4.

15. Procédé selon la revendication 1, caractérisé en ce qu'on pr prépare le mélange homogène par formation d'un produit de lyophilisation.

16. Procédé selon la revendication 1, caracterisé en ce qu'on procède à la dispersion à des températures inférieures à 30°.

17. Procédé selon la revendication 12, caractérisé en ce qu'on utilise pour la préparation du produit de lyophilisation le tert-butanol comme solvant.

18. Mélanges constitués de phospholipides synthétiques de formules I et II purs a plus de 90% en poids selon la revendication 1.

**Claims for the Contracting States: BE CH DE FR GB IT LI LU NL SE**

1. Pharmaceutical compositions containing

a) a synthetic, more than 90% by weight pure phospholipid of the formula

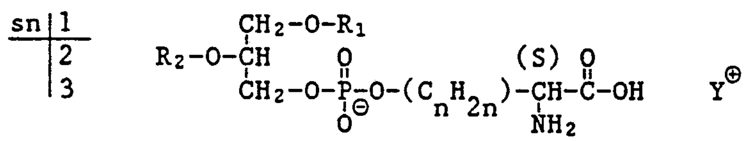

(I),

in which $R_1$ and $R_2$ each represents, independently of the other, $C_{10}$—$C_{20}$-alkenoyl having an even number of carbon atoms, *n* represents an integer from one to three and $Y^{\oplus}$ represents the cation of a pharmaceutically acceptable base,

b) a synthetic, more than 90% by weight pure phospholipid of the formula

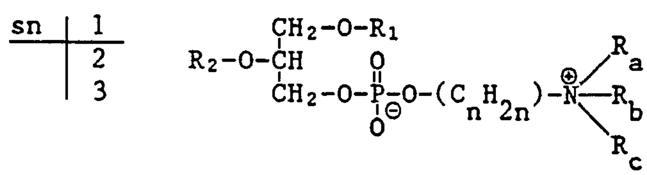

(II) ,

in which $R_1$ represents $C_{10}$—$C_{20}$-alkanoyl having an even number of carbon atoms, $R_2$ represents $C_{10}$—$C_{20}$-alkenoyl having an even number of carbon atoms, $R_a$, $R_b$ and $R_c$ represent hydrogen or $C_1$—$C_4$-alkyl and *n* represents an integer from two to four,

c) a substance or a mixture of substances having biological activity, and optionally a carrier liquid and/or additional solid carriers.

2. Pharmaceutical compositions according to claim 1 containing synthetic, more than 90% by weight pure phospholipids in a mixing ratio of phospholipids of the formula I to phospholipids of the formula II of from approximately 10:90 to approximately 50:50 mol%, and a pharmaceutically active substance or a mixture of pharmaceutically active substances, and optionally a carrier liquid buffered to pH 7.0—7.8 and/or additional solid carriers.

3. Pharmaceutical compositions according to claim 1 or 2 containing

a) a synthetic, more than 90% by weight pure phospholipid of the formula I in which $R_1$ and $R_2$ are the same and represent 9-*cis*-dodecenoyl, 9-*cis*-tetradecenoyl, 9-*cis*-hexadecenoyl, 9-*cis*-octadecenoyl or 9-*cis*-icosenoyl, *n* is one and $Y^{\oplus}$ is the sodium ion,

b) a synthetic, more than 90% by weight pure phospholipid of the formula II in which $R_1$ represents n-dodecanoyl, n-tetradecanoyl, n-hexadecanoyl or n-octadecanoyl, $R_2$ represents 9-*cis*-dodecenoyl, 9-*cis*-tetradecenoyl, 9-*cis*-hexadecenoyl, 9-*cis*-octadecenoyl or 9-*cis*-icosenoyl and $R_a$, $R_b$ and $R_c$ represent methyl,

c) a pharmaceutically active substance or a mixture of pharmaceutically active substances, and optionally a carrier liquid buffered to pH 7.2—7.4.

4. Pharmaceutical compositions according to claim 3 containing

a) synthetic, more than 90% by weight pure sodium 1,2 - di - (9 - *cis* - octadecenoyl) - 3 - sn - phosphatidyl - (S) - serine,

b) synthetic, more than 90% by weight pure 1 - n - hexadecanoyl - 2 - (9 - *cis* - octadecenoyl) - 3 - sn - phosphatidyl choline,

c) a pharmaceutically active substance or a mixture of pharmaceutically active substances, and optionally a carrier liquid buffered to pH 7.2—7.4.

5. Pharmaceutical compositions according to claim 1 containing synthetic, more than 90% by weight pure phospholipids in a mixing ratio of phospholipids of the formula I to phospholipids of the formula II of from approximately 10:90 to approximately 50:50 mol%, and a pharmaceutically active substance or a mixture of substances selected from the group consisting of antiphlogistics, antibiotics, antileishmaniasis agents, antimycotics, antineoplastics and immunomodulators, and optionally a carrier liquid buffered to pH 7.0—7.8 and/or additional solid carriers.

6. Pharmaceutical compositions according to claim 1 or 2 containing

a) a synthetic, more than 90% by weight pure phospholipid of the formula I in which $R_1$ and $R_2$ are the same and represent 9-*cis*-dodecenoyl, 9-*cis*-tetradecenoyl, 9-*cis*-hexadecenoyl, 9-*cis*-octadecenoyl or 9-*cis*-icosenoyl, *n* is one and $Y^{\oplus}$ is the sodium ion,

b) a synthetic, more than 90% by weight pure phospholipid of the formula II in which $R_1$ represents n-dodecanoyl, n-tetradecanoyl, n-hexadecanoyl or n-octadecenoyl, $R_2$ represents 9-*cis*-dodecenoyl, 9-*cis*-tetradecenoyl, 9-*cis*-hexadecenoyl, 9-*cis*-octadecenoyl or 9-*cis*-icosenoyl and $R_a$, $R_b$ and $R_c$ represent methyl,

c) a pharmaceutically active substance or a mixture of substances selected from the group consisting of antiphlogistics, antibiotics, antileishmaniasis agents, antimycotics, antineoplastics and immunomodulators, and optionally a carrier liquid buffered to pH 7.0—7.4.

7. Pharmaceutical compositions according to claim 3 containing

a) synthetic, more than 90% by weight pure sodium 1,2 - di - (9 - *cis* - octadecenoyl) - 3 - sn - phosphatidyl - (S) - serine,

b) synthetic, more than 90% by weight pure 1 - n - hexadecanoyl - 2 - (9 - *cis* - octadecenoyl) - 3 - sn - phosphatidyl choline,

c) a pharmaceutically active substance or a mixture of substances selected from the group consisting of antiphlogistics, antibiotics, antileishmaniasis agents, antimycotics, antineoplastics and immunomodulators, and optionally a carrier liquid buffered to pH 7.0—7.4.

8. Pharmaceutical compositions according to claim 4 containing

a) synthetic, more than 90% by weight pure sodium 1,2 - di - (9 - *cis* - octadecenoyl) - 3 - sn - phosphatidyl - (S) - serine,

b) synthetic, more than 90% by weight pure 1 - n - hexadecanoyl - 2 - (9 - *cis* - octadecenoyl) - 3 - sn - phosphatidyl choline,

c) a pharmaceutically active substance or a mixture of substances selected from the group consisting of diclofenac, pirprofen, mitomycin, cytarabine, dactinomycin, daunorubicin, doxorubicin, etoposide, N - acetyl - D - muramyl - L - alanyl - D - isoglutaminyl - L - alanine - 2 - (1,2 - dipalmitoyl) - sn - glycero - 3 - hydroxyphosphoryloxy) - ethylamide, the disodium salt of N - acetyl - D - muramyl - L - alanyl - D - glutamic acid-($C_\gamma$) - L - alanine - 2 - (1,2 - dipalmitoyl - sn - glycero - 3 - hydroxyphosphoryloxy) - ethylamide, the sodium salt of N - acetyldesmethylmuramyl - L - alanyl - D - isoglutamine, the sodium salt of N - acetyl - D - muramyl - L - alanyl - D - isoglutamine, N - acetyl - D - muramyl - L - alanyl - D - glutamine - α - n - butyl ester, $N^\alpha$ - (N - acetyl - D - muramyl - L - alanyl - D - isoglutaminyl) - $N^\varepsilon$-stearoyl - L - lysin or 6 - O - stearoyl - N - acetyl - D - muramyl - L - alanine - D - isoglutamine, lymphoketone or combinations thereof, and optionally a carrier liquid buffered to pH 7.2—7.4.

9. Pharmaceutical compositions according to claim 4 containing

a) synthetic, more than 90% by weight pure sodium 1,2 - di - (9 - *cis* - octadecenoyl) - 3 - sn - phosphatidyl - (S) - serine,

b) synthetic, more than 90% by weight pure 1 - n - hexadecanoyl - 2 - (9 - *cis* - octadecenoyl) - 3 - sn - phosphatidyl choline,

c) a pharmaceutically active substance selected from the group consisting of mitomycin, cytarabine, dactinomycin, daunorubicin, doxorubicin, etoposide, N - acetyl - D - muramyl - L - alanyl - D - isoglutaminyl - L - alanine - 2 - (1,2 - dipalmitoyl) - sn - glycero - 3 - hydroxyphosphoryloxy) - ethylamide, the sodium salt of N - acetyldesmethylmuramyl - L - alanyl - D - isoglutamine or the sodium salt of N - acetyl - D - muramyl - L - alanyl - D - isoglutamine, optionally in combination with human gamma-interferon or interleukin 2 or mixtures of substances that are contained in culture filtrates obtained from cultures having human T-lymphocytes from the spleen or peripheral blood after stimulation by antigens or mitogens and that are characterised by a high content of macrophage-activating factor (MAF), and optionally a carrier liquid buffered to pH 7.2—7.4.

10. Pharmaceutical compositions according to claim 4 containing

a) synthetic, more than 90% by weight pure sodium 1,2 - di - (9 - *cis* - octadecenoyl) - 3 - sn - phosphatidyl - (S) - serine,

b) synthetic, more than 90% by weight pure 1 - n - hexadecanoyl - 2 - (9 - *cis* - octadecenoyl) - 3 - sn - phosphatidyl choline,

c) N - acetyl - D - muramyl - L - alanyl - D - isoglutaminyl - L - alanine - 2 - (1,2 - dipalmitoyl) - sn - glycero - 3 - hydroxyphosphoryloxy) - ethylamide, the sodium salt of N - acetyldesmethylmuramyl - L - alanyl - D - isoglutamine or the sodium salt of N - acetyl - D - muramyl - L - alanyl - D - isoglutamine, optionally in combination with mixtures of substances that are contained in culture filtrates from cultures having human T-lymphocytes from the spleen or peripheral blood after stimulation by antigens or mitogens and that are characterised by at least 70% content of MAF, and optionally a carrier liquid buffered to pH 7.2—7.4.

11. Pharmaceutical compositions according to claim 4 containing

a) synthetic, more than 90% by weight pure sodium 1,2 - di - (9 - *cis* - octadecenoyl) - 3 - sn - phosphatidyl - (S) - serine,

b) synthetic, more than 90% by weight pure 1 - n - hexadecanoyl - 2 - (9 - *cis* - octadecenoyl) - 3 - sn - phosphatidyl choline,

c) N - acetyl - D - muramyl - L - alanyl - D - isoglutaminyl - L - alanine - 2 - (1,2 - dipalmitoyl) - sn - glycero - 3 - hydroxyphosphoryloxy) - ethylamide, and optionally a carrier liquid buffered to pH 7.2—7.4.

12. Pharmaceutical compositions according to claim 4 containing

a) synthetic, more than 90% by weight pure sodium 1,2 - di - (9 - *cis* - octadecenoyl) - 3 - sn - phosphatidyl - (S) - serine,

b) synthetic, more than 90% by weight pure 1 - n - hexadecanoyl - 2 - (9 - *cis* - octadecenoyl) - 3 - sn - phosphatidyl choline,

c) natural or recombinant human gamma-interferon and optionally a carrier liquid buffered to pH 7.2—7.4.

13. Pharmaceutical compositions according to claim 9 containing

a) synthetic, more than 90% by weight pure sodium 1,2 - di - (9 - *cis* - octadecenoyl) - 3 - sn - phosphatidyl - (S) - serine,

b) synthetic, more than 90% by weight pure 1 - n - hexadecanoyl - 2 - (9 - *cis* - octadecenoyl) - 3 - sn - phosphatidyl choline,

c) recombinant human gamma-interferon having the following amino acid sequence:

$H_2N$-Cys-Tyr-Cys-Gln-Asp-Pro-Tyr-Val-Gln-Glu-Ala-Glu-Asn-Leu-Lys-Lys-
Tyr-Phe-Asn-Ala-Gly-His-Ser-Asp-Val-Ala-Asp-Asn-Gly-Thr-Leu-Phe-Leu-
Gly-Ile-Leu-Lys-Asn-Trp-Lys-Glu-Glu-Ser-Asp-Arg-Lys-Ile-Met-Gln-Ser-
Gln-Ile-Val-Ser-Phe-Tyr-Phe-Lys-Leu-Phe-Lys-Asn-Phe-Lys-Asp-Asp-Gln-
Ser-Ile-Gln-Lys-Ser-Val-Glu-Thr-Ile-Lys-Glu-Asp-Met-Asn-Val-Lys-Phe-
Phe-Asn-Ser-Asn-Lys-Lys-Lys-Arg-Asp-Asp-Phe-Glu-Lys-Leu-Thr-Asn-Tyr-
Ser-Val-Thr-Asp-Leu-Asn-Val-Gln-Arg-Lys-Ala-Ile-His-Glu-Leu-Ile-Gln-
Val-Met-Ala-Glu-Leu-Ser-Pro-Ala-Ala-Lys-Thr-Glu-Lys-Arg-Lys-Arg-Ser-
Gln-Met-Leu-Phe-Gln-Gly-Arg-Arg-Ala-Ser-Gln-OH

and optionally a carrier liquid buffered to pH 7.2—7.4.

14. Pharmaceutical compositions according to claim 9 containing

a) synthetic, more than 90% by weight pure sodium 1,2 - di - (9 - *cis* - octadecenoyl) - 3 - sn - phosphatidyl - (S) - serine,

b) synthetic, more than 90% by weight pure 1 - n - hexadecanoyl - 2 - (9 - *cis* - octadecenoyl) - 3 - sn - phosphatidyl choline,

c) recombinant human gamma-interferon having the following amino acid sequence:

$H_2N$-Cys-Tyr-Cys-Gln-Asp-Pro-Tyr-Val-Lys-Glu-Ala-
Glu-Asn-Leu-Lys-Lys-Tyr-Phe-Asn-Ala-Gly-His-Ser-
Asp-Val-Ala-Asp-Asn-Gly-Thr-Leu-Phe-Leu-Gly-Ile-
Leu-Lys-Asn-Trp-Lys-Glu-Glu-Ser-Asp-Arg-Lys-Ile-
Met-Gln-Ser-Gln-Ile-Val-Ser-Phe-Tyr-Phe-Lys-Leu-
Phe-Lys-Asn-Phe-Lys-Asp-Asp-Gln-Ser-Ile-Gln-Lys-
Ser-Val-Glu-Thr-Ile-Lys-Glu-Asp-Met-Asn-Val-Lys-
Phe-Phe-Asn-Ser-Asn-Lys-Lys-Lys-Arg-Asp-Asp-Phe-
Glu-Lys-Leu-Thr-Asn-Tyr-Ser-Val-Thr-Asp-Leu-Asn-
Val-Gln-Arg-Lys-Ala-Ile-His-Glu-Leu-Ile-Gln-Val-
Met-Ala-Glu-Leu-Ser-Pro-Ala-Ala-Lys-Thr-Gly-Lys-
Arg-Lys-Arg-Ser-Gln-Met-Leu-Phe-Arg-Gly-Arg-Arg-
Ala-Ser-Gln-OH

and optionally a carrier liquid buffered to pH 7.2—7.4.

15. Mixtures suitable for the manufacture of liposomes comprising synthetic, more than 90% by weight pure phospholipids of the formulae I and II according to claim 1.

16. A process for the manufacture of pharmaceutical compositions according to any one of claims 1 to 11, which comprises

a) manufacturing a homogeneous mixture consisting of synthetic, more than 90% by weight pure phospholipids of the formulae I and II and a lipophilic substance or mixture of substances having biological activity, and, for the manufacture of liposomes, dispersing the obtainable homogeneous mixture in an aqueous phase, or

b) manufacturing a homogeneous mixture consisting of synthetic, more than 90% by weight pure phospholipids of the formulae I and II, and, for the manufacture of liposomes, dispersing the obtainable homogeneous mixture in an aqueous phase containing a water-soluble substance or mixture of substances having biological activity, and, if necessary, buffering the obtainable aqueous dispersion to pH 7.0—7.8 and, if desired, enriching and/or separating off the obtainable liposomes.

17. A pharmaceutical composition according to claim 1 for use in the treatment of the human or animal body.

**Claims for the Contracting State: AT**

1. A process for the manufacture of pharmaceutical compositions containing

a) a synthetic, more than 90% by weight pure phospholipid of the formula

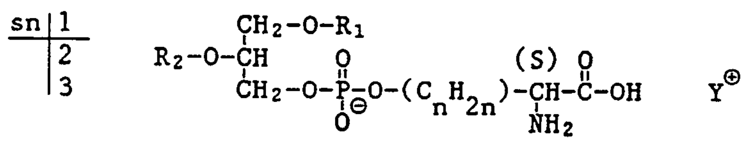 (I),

in which $R_1$ and $R_2$ each represents, independently of the other, $C_{10}$—$C_{20}$-alkenoyl having an even number of carbon atoms, *n* represents an integer from one to three and $Y^{\oplus}$ represents the cation of a pharmaceutically acceptable base,

b) a synthetic, more than 90% by weight pure phospholipid of the formula

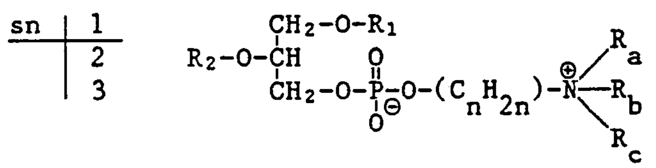 (II) ,

in which $R_1$ represents $C_{10}$—$C_{20}$-alkanoyl having an even number of carbon atoms, $R_2$ represents $C_{10}$—$C_{20}$-alkenoyl having an even number of carbon atoms, $R_a$, $R_b$ and $R_c$ represent hydrogen or $C_1$—$C_4$-alkyl and *n* represents an integer from two to four,

c) a substance or a mixture of substances having biological activity, and optionally a carrier liquid and/or additional solid carriers, which process comprises

a) manufacturing a homogeneous mixture consisting of synthetic, more than 90% by weight pure phospholipids of the formulae I and II and a lipophilic substance or mixture of substances having biological activity, and, for the manufacture of liposomes, dispersing the obtainable homogeneous mixture in an aqueous phase, or

b) manufacturing a homogeneous mixture consisting of synthetic, more than 90% by weight pure phospholipids of the formulae I and II, and, for the manufacture of liposomes, dispersing the obtainable homogeneous mixture in an aqueous phase containing a water-soluble substance or mixture of substances having biological activity, and, if necessary, buffering the obtainable aqueous dispersion to pH 7.0—7.8 and, if desired, enriching and/or separating off the obtainable liposomes.

2. A process for the manufacture of pharmaceutical compositions according to claim 1 containing synthetic, more than 90% by weight pure phospholipids in a mixing ratio of phospholipids of the formula I to phospholipids of the formula II of from approximately 10:90 to approximately 50:50 mol%, and a pharmaceutically active substance or a mixture of pharmaceutically active substances, and optionally a carrier liquid buffered to pH 7.0—7.8 and/or additional solid carriers, which comprises carrying out the measures mentioned in claim 1.

3. A process for the manufacture of pharmaceutical compositions according to claim 1 or 2 containing

a) a synthetic, more than 90% by weight pure phospholipid of the formula I in which $R_1$ and $R_2$ are the same and represent 9-*cis*-dodecenoyl, 9-*cis*-tetradecenoyl, 9-*cis*-hexadecenoyl, 9-*cis*-octadecenoyl or 9-*cis*-icosenoyl, *n* is one and $Y^{\oplus}$ is the sodium ion,

b) a synthetic, more than 90% by weight pure phospholipid of the formula II in which $R_1$ represents

n-dodecanoyl, n-tetradecanoyl, n-hexadecanoyl or n-octadecanoyl, $R_2$ represents 9-*cis*-dodecenoyl, 9-*cis*-tetradecenoyl, 9-*cis*-hexadecenoyl, 9-*cis*-octadecenoyl or 9-*cis*-icosenoyl and $R_a$, $R_b$ and $R_c$ represent methyl,

c) a pharmaceutically active substance or a mixture of pharmaceutically active substances, and optionally a carrier liquid buffered to pH 7.2—7.4, which comprises carrying out the measures mentioned in claim 1.

4. A process for the manufacture of pharmaceutical compositions according to claim 3 containing

a) synthetic, more than 90% by weight pure sodium 1,2 - di - (9 - *cis* - octadecenoyl) - 3 - sn - phosphatidyl - (S) - serine,

b) synthetic, more than 90% by weight pure 1 - n - hexadecanoyl - 2 - (9 - *cis* - octadecenoyl) - 3 - sn - phosphatidyl choline,

c) a pharmaceutically active substance or a mixture of pharmaceutically active substances, and optionally a carrier liquid buffered to pH 7.2—7.4, which comprises carrying out the measures mentioned in claim 1.

5. A process for the manufacture of pharmaceutical compositions according to claim 1 containing synthetic, more than 90% by weight pure phospholipids in a mixing ratio of phospholipids of the formula I to phospholipids of the formula II of from approximately 10:90 to approximately 50:50 mol%, and a pharmaceutically active substance or a mixture of substances selected from the group consisting of antiphlogistics, antibiotics, antileishmaniasis agents, antimycotics, antineoplastics and immunomodulators, and optionally a carrier liquid buffered to pH 7.0—7.8 and/or additional solid carriers, which comprises carrying out the measures mentioned in claim 1.

6. A process for the manufacture of pharmaceutical compositions according to claim 1 or 2 containing

a) a synthetic, more than 90% by weight pure phospholipid of the formula I in which $R_1$ and $R_2$ are the same and represent 9-*cis*-dodecenoyl, 9-*cis*-tetradecenoyl, 9-*cis*-hexadecenoyl, 9-*cis*-octadecenoyl or 9-*cis*-icosenoyl, *n* is one and $Y^{\oplus}$ is the sodium ion,

b) a synthetic, more than 90% by weight pure phospholipid of the formula in which $R_1$ represents n-dodecanoyl, n-tetradecanoyl, n-hexadecanoyl or n-octadecenoyl, $R_2$ represents 9-*cis*-dodecenoyl, 9-*cis*-tetradecenoyl, 9-*cis*-hexadecenoyl, 9-*cis*-octadecenoyl or 9-*cis*-icosenoyl and $R_a$, $R_b$ and $R_c$ represent methyl,

c) a pharmaceutically active substance or a mixture of substances selected from the group consisting of antiphlogistics, antibiotics, antileishmaniasis agents, antimycotics, antineoplastics and immunomodulators, and optionally a carrier liquid buffered to pH 7.0—7.4, which comprises carrying out the measures mentioned in claim 1.

7. A process for the manufacture of pharmaceutical compositions according to claim 3 containing

a) synthetic, more than 90% by weight pure sodium 1,2 - di - (9 - *cis* - octadecenoyl) - 3 - sn - phosphatidyl - (S) - serine,

b) synthetic, more than 90% by weight pure 1 - n - hexadecanoyl - 2 - (9 - *cis* - octadecenoyl) - 3 - sn - phosphatidyl choline,

c) a pharmaceutically active substance or a mixture of substances selected from the group consisting of antiphlogistics, antibiotics, antileishmaniasis agents, antimycotics, antineoplastics and immunomodulators, and optionally a carrier liquid buffered to pH 7.0—7.4, which comprises carrying out the measures mentioned in claim 1.

8. A process for the manufacture of pharmaceutical compositions according to claim 4 containing

a) synthetic, more than 90% by weight pure sodium 1,2 - di - (9 - *cis* - octadecenoyl) - 3 - sn - phosphatidyl - (S) - serine,

b) synthetic, more than 90% by weight pure 1 - n - hexadecanoyl - 2 - (9 - *cis* - octadecenoyl) - 3 - sn - phosphatidyl choline,

c) diclofenac, pirprofen, mitomycin, cytarabine, dactinomycin, daunorubicin, doxorubicin, etoposide, N - acetyl - D - muramyl - L - alanyl - D - isoglutaminyl - L - alanine - 2 - (1,2 - dipalmitoyl) - sn - glycero - 3 - hydroxyphosphoryloxy) - ethylamide, the disodium salt of N - acetyl - D - muramyl - L - alanyl - D - glutamic acid-($C_{\gamma}$) - L - alanine - 2 - (1,2 - dipalmitoyl - sn - glycero - 3 - hydroxyphosphoryloxy) - ethylamide, the sodium salt of N - acetyldesmethylmuramyl - L - alanyl - D - isoglutamine, the sodium salt of N - acetyl - D - muramyl - L - alanyl - D - isoglutamine, N - acetyl - D - muramyl - L - alanyl - D - glutamine - α - n - butyl ester, $N^{\alpha}$ - (N - acetyl - D - muramyl - L - alanyl - D - isoglutaminyl) - $N^{\varepsilon}$-stearoyl - L - lysin or 6 - O - stearoyl - N - acetyl - D - muramyl - L - alanine - D - isoglutamine, lymphokines or combinations thereof, and optionally a carrier liquid buffered to pH 7.2—7.4, which comprises carrying out the measures mentioned in claim 1.

9. A process for the manufacture of pharmaceutical compositions according to claim 5 containing

a) synthetic, more than 90% by weight pure sodium 1,2 - di - (9 - *cis* - octadecenoyl) - 3 - sn - phosphatidyl - (S) - serine,

b) synthetic, more than 90% by weight pure 1 - n - hexadecanoyl - 2 - (9 - *cis* - octadecenoyl) - 3 - sn - phosphatidyl choline,

c) mitomycin, cytarabine, dactinomycin, daunorubicin, doxorubicin, etoposide, N - acetyl - D - muramyl - L - alanyl - D - isoglutaminyl - L - alanine - 2 - (1,2 - dipalmitoyl) - sn - glycero - 3 - hydroxyphosphoryloxy) - ethylamide, the sodium salt of N - acetyldesmethylmuramyl - L - alanyl - D -

isoglutamine or the sodium salt of N - acetyl - D - muramyl - L - alanyl - D - isoglutamine, optionally in combination with human gamma-interferon or interleukin 2 or mixtures of substances that are contained in culture filtrates obtained from cultures having human T-lymphocytes from the spleen or peripheral blood after stimulation by antigens or mitogens and that are characterised by a high content of macrophage-activating factor (MAF), and optionally a carrier liquid buffered to pH 7.2—7.4, which comprises carrying out the measures mentioned in claim 1.

10. Pharmaceutical compositions according to claim 6 containing

a) synthetic, more than 90% by weight pure sodium 1,2 - di - (9 - *cis* - octadecenoyl) - 3 - sn - phosphatidyl - (S) - serine,

b) synthetic, more than 90% by weight pure 1 - n - hexadecanoyl - 2 - (9 - *cis* - octadecenoyl) - 3 - sn - phosphatidyl choline,

c) N - acetyl - D - muramyl - L - alanyl - D - isoglutaminyl - L - alanine - 2 - (1,2 - dipalmitoyl - sn - glycero - 3 - hydroxyphosphoryloxy) - ethylamide, the sodium salt of N - acetyldesmethylmuramyl - L - alanyl - D - isoglutamine or the sodium salt of N - acetyl - D - muramyl - L - alanyl - D - isoglutamine, optionally in combination with mixtures of substances that are contained in culture filtrates obtained from cultures having human T-lymphocytes from the spleen or peripheral blood after stimulation by antigens or mitogens and that are characterised by at least 70% content of MAF, and optionally a carrier liquid buffered to pH 7.2—7.4, which comprises carrying out the measures mentioned in claim 1.

11. A process for the manufacture of pharmaceutical compositions according to claim 4 containing

a) synthetic, more than 90% by weight pure sodium 1,2 - di - (9 - *cis* - octadecenoyl) - 3 - sn - phosphatidyl - (S) - serine,

b) synthetic, more than 90% by weight pure 1 - n - hexadecanoyl - 2 - (9 - *cis* - octadecenoyl) - 3 - sn - phosphatidyl choline,

c) N - acetyl - D - muramyl - L - alanyl - D - isoglutaminyl - L - alanine - 2 - (1,2 - dipalmitoyl) - sn - glycero - 3 - hydroxyphosphoryloxy) - ethylamide, and optionally a carrier liquid buffered to pH 7.2—7.4, which comprises carrying out the measures mentioned in claim 1.

12. A process for the manufacture of pharmaceutical compositions according to claim 4 containing

a) synthetic, more than 90% by weight pure sodium 1,2 - di - (9 - *cis* - octadecenoyl) - 3 - sn - phosphatidyl - (S) - serine,

b) synthetic, more than 90% by weight pure 1 - n - hexadecanoyl - 2 - (9 - *cis* - octadecenoyl) - 3 - sn - phosphatidyl choline,

c) natural or recombinant human gamma-interferon and optionally a carrier liquid buffered to pH 7.2—7.4, which comprises carrying out the measures mentioned in claim 1.

13. A process for the manufacture of pharmaceutical compositions according to claim 9 containing

a) synthetic, more than 90% by weight pure sodium 1,2 - di - (9 - *cis* - octadecenoyl) - 3 - sn - phosphatidyl - (S) - serine,

b) synthetic, more than 90% by weight pure 1 - n - hexadecanoyl - 2 - (9 - *cis* - octadecenoyl) - 3 - sn - phosphatidyl choline,

c) recombinant human gamma-interferon having the following amino acid sequence:

$H_2N$-Cys-Tyr-Cys-Gln-Asp-Pro-Tyr-Val-Gln-Glu-Ala-Glu-Asn-Leu-Lys-Lys-
Tyr-Phe-Asn-Ala-Gly-His-Ser-Asp-Val-Ala-Asp-Asn-Gly-Thr-Leu-Phe-Leu-
Gly-Ile-Leu-Lys-Asn-Trp-Lys-Glu-Glu-Ser-Asp-Arg-Lys-Ile-Met-Gln-Ser-
Gln-Ile-Val-Ser-Phe-Tyr-Phe-Lys-Leu-Phe-Lys-Asn-Phe-Lys-Asp-Asp-Gln-
Ser-Ile-Gln-Lys-Ser-Val-Glu-Thr-Ile-Lys-Glu-Asp-Met-Asn-Val-Lys-Phe-
Phe-Asn-Ser-Asn-Lys-Lys-Lys-Arg-Asp-Asp-Phe-Glu-Lys-Leu-Thr-Asn-Tyr-
Ser-Val-Thr-Asp-Leu-Asn-Val-Gln-Arg-Lys-Ala-Ile-His-Glu-Leu-Ile-Gln-
Val-Met-Ala-Glu-Leu-Ser-Pro-Ala-Ala-Lys-Thr-Glu-Lys-Arg-Lys-Arg-Ser-
Gln-Met-Leu-Phe-Gln-Gly-Arg-Arg-Ala-Ser-Gln-OH

and optionally a carrier liquid buffered to pH 7.2—7.4, which comprises carrying out the measures mentioned in claim 1.

14. A process for the manufacture of pharmaceutical compositions according to claim 9 containing

a) synthetic, more than 90% by weight pure sodium 1,2 - di - (9 - *cis* - octadecenoyl) - 3 - sn - phosphatidyl - (S) - serine,

b) synthetic, more than 90% by weight pure 1 - n - hexadecanoyl - 2 - (9 - *cis* - octadecenoyl) - 3 - sn - phosphatidyl choline,

c) recombinant human gamma-interferon having the following amino acid sequence:

$H_2N$-Cys-Tyr-Cys-Gln-Asp-Pro-Tyr-Val-Lys-Glu-Ala-
Glu-Asn-Leu-Lys-Lys-Tyr-Phe-Asn-Ala-Gly-His-Ser-
Asp-Val-Ala-Asp-Asn-Gly-Thr-Leu-Phe-Leu-Gly-Ile-
Leu-Lys-Asn-Trp-Lys-Glu-Glu-Ser-Asp-Arg-Lys-Ile-
Met-Gln-Ser-Gln-Ile-Val-Ser-Phe-Tyr-Phe-Lys-Leu-
Phe-Lys-Asn-Phe-Lys-Asp-Asp-Gln-Ser-Ile-Gln-Lys-
Ser-Val-Glu-Thr-Ile-Lys-Glu-Asp-Met-Asn-Val-Lys-
Phe-Phe-Asn-Ser-Asn-Lys-Lys-Lys-Arg-Asp-Asp-Phe-
Glu-Lys-Leu-Thr-Asn-Tyr-Ser-Val-Thr-Asp-Leu-Asn-
Val-Gln-Arg-Lys-Ala-Ile-His-Glu-Leu-Ile-Gln-Val-
Met-Ala-Glu-Leu-Ser-Pro-Ala-Ala-Lys-Thr-Gly-Lys-
Arg-Lys-Arg-Ser-Gln-Met-Leu-Phe-Arg-Gly-Arg-Arg-
Ala-Ser-Gln-OH

and optionally a carrier liquid buffered to pH 7.2—7.4, which comprises carrying out the measures mentioned in claim 1.

15. A process according to claim 1, which comprises manufacturing the homogeneous mixture by the formation of a lyophilisate.

16. A process according to claim 1, which comprises carrying out the dispersion at temperatures below 30°.

17. A process according to claim 12, which comprises using tert.-butanol as solvent for the manufacture of the lyophilisate.

18. Mixtures comprising synthetic, more than 90% by weight pure phospholipids of the formulae I and II according to claim 1.